# EUROPÄISCHE PATENTSCHRIFT

(11) **EP 3 854 374 B1**
(45) Veröffentlichungstag und Bekanntmachung des Hinweises auf die Patenterteilung: **01.07.2026**
(21) Anmeldenummer: 20153683.6
(22) Anmeldetag: 24.01.2020
(51) Int. Cl.: A61K 6/16, A61K 6/17, A61K 6/62, A61K 6/71, A61K 6/76, A61K 6/77, A61K 6/818, A61K 6/887

(54) **ÄSTHETISCHES DENTALES FÜLLUNGSMATERIAL MIT HOHER DURCHHÄRTUNGSTIEFE**
AESTHETIC DENTAL FILLING MATERIAL WITH HIGH CURING DEPTH
MATIÈRE DE REMPLISSAGE DENTAIRE ESTHÉTIQUE À PROFONDEUR DE DURCISSEMENT ÉLEVÉE

(43) Veröffentlichungstag der Anmeldung: 28.07.2021
(73) Patentinhaber: Ivoclar Vivadent AG, 9494 Schaan (LI)
(72) Erfinder: Busch, Susanne, 6820 Frastanz (AT); GEBHARDT, Benjamin, 9472 Grabs (CH)
(74) Vertreter: Uexküll & Stolberg

(56) Entgegenhaltungen:
- EP-A1- 2 965 741
- WO-A1-2016/026915
- US-A1- 2010 035 214
- US-A1- 2019 192 386
- NORBERT MOSZNER ET AL: "New Diluents for Dental Composites", MACROMOLECULAR MATERIALS AND ENGINEERING., vol. 301, no. 6, 25 April 2016 (2016-04-25), DE, pages 750 - 759, XP055706026, ISSN: 1438-7492, DOI: 10.1002/mame.201600115

## Beschreibung

Die vorliegende Erfindung betrifft röntgenopake Dentalwerkstoffe, die sich durch eine große Durchhärtungstiefe auszeichnen und eine vereinfachte Herstellung ästhetisch ansprechender Dentalrestaurationen erlauben. Die Werkstoffe eignen sich insbesondere als dentale Füllungsmaterialien.

Der Dentalmarkt bietet eine kaum noch zu überschauende Anzahl an Füllungsmaterialien für alle denkbaren Indikationen in der Füllungstherapie. Die Entwicklung im Bereich der methacrylatbasierten Füllungsmaterialien hat mittlerweile ein so hohes Niveau erreicht, dass ein professionell restaurierter Zahn praktisch nicht mehr von seinem natürlichen Vorbild unterschieden werden kann. Dies macht es schwierig, zwischen der Restauration und der natürlichen Zahnsubstanz zu differenzieren, was insbesondere im Hinblick auf spätere Behandlungen nachteilig ist. Es besteht daher Bedarf an Dentalwerkstoffen, die neben einer hohen Ästhetik eine hohe Röntgenopazität aufweisen und die so eine eindeutige Unterscheidung von der natürlichen Zahnsubstanz ermöglichen.

Die Herstellung ästhetisch ansprechender Restaurationen ist mit einem hohen Aufwand für den Zahnarzt verbunden. Gegenwärtig werden für eine ästhetische Füllung im Allgemeinen zwei bis vier unterschiedliche Massen verwendet, um das natürliche Erscheinungsbild der verlorenen Zahnhartsubstanz möglichst naturgetreu nachzuahmen. Um die Vielfalt der natürlichen Zahnfarben abzubilden, werden Farbpaletten mit 30 und mehr unterschiedlichen Farben in diversen Opazitäten angeboten, aus denen die für den jeweiligen Behandlungsfall optimale Materialkombination ausgewählt werden muss. Es wäre wünschenswert, Materialen zur Verfügung zu haben, welche die Herstellung ästhetisch ansprechender Restaurationen mit einem geringeren Materialaufwand ermöglichen.

Dentale Füllungsmaterialien auf Methacrylatbasis werden oft als Kunststofffüllungen oder korrekter als Komposite bezeichnet. Kompositmaterialien enthalten eine polymerisierbare organische Matrix und Füllstoffe sowie diverse Zuschlagsstoffe, wie Stabilisatoren, Initiatoren und Pigmenten. Der Füllstoffgehalt hängt maßgeblich vom gewünschten Verwendungszweck ab und kann bis zu 90 Gew.-% betragen.

Die polymerisierbare organische Matrix von dentalen Füllungskompositen und Adhäsiven basiert meist auf einer Mischung von Dimethacrylaten, die meist das hochviskose BisGMA als Vernetzer enthalten. BisGMA führt zu guten mechanischen Eigenschaften bei vergleichsweise geringem Schrumpf. Allerdings enthält kommerziell erhältliches BisGMA häufig Bis-Phenol-A als Verunreinigung. Weitere Beispiele für häufig eingesetzte Dimethacrylate sind Urethandimethacrylate und die regelmäßig als Verdünnermonomere genutzten, niedrigviskosen Dimethacrylate Bis(methacryl-oyloxymethyl)tricyclo[5.2.1.]decan (TCDMA), Decandiol-1,10-dimethacrylat (D₃MA) und Triethylenglycoldimethacrylat (TEGDMA).

Die Materialen enthalten in der Regel einen Initiator für die radikalische Polymerisation, wobei lichthärtende Werkstoffe, die einen Photoinitiator enthalten, heutzutage in der zahnärztlichen Füllungstherapie eine dominante Stellung einnehmen. Ein Nachteil von lichthärtenden Materialien ist, dass besonders das Legen großer Füllungen aufwändig ist, weil das zur Härtung erforderliche Licht nur bis zu einer begrenzten Tiefe in die Materialien eindringen kann. Bei der sogenannten Inkrementtechnik wird die Füllung daher schichtweise aus dem Kompositwerkstoff aufgebaut, wobei die Schichten eine Dicke von jeweils ca. 2 mm haben und einzeln gehärtet werden müssen.

Diesen Nachteil überwinden sogenannte Bulk-Fill-Materialien, die Durchhärtungstiefen von ca. 4 mm pro Schicht erlauben. Diese Materialien verfügen jedoch oft nicht über die gewünschten ästhetischen Eigenschaften und sind daher nicht oder nur eingeschränkt für die Frontzahnrestauration geeignet. Die Durchhärtungstiefe korreliert mit der Transluzenz der Werkstoffe, wobei eine hohe Transluzenz und eine gute Durchhärtungstiefe dann erreicht werden, wenn die organische Matrix und die verwendeten Füllstoffe übereinstimmende Brechungsindices aufweisen. Nachteilig hierbei ist, dass solche Komposite aufgrund ihrer hohen Transluzenz das darunterliegende Dentin nur schlecht abdecken, was aus ästhetischen Gründen störend ist, weil die Farbe des Dentins von der des sichtbaren Zahnschmelzes abweicht.

Die WO 2016/026915 A1 offenbart radikalisch polymerisierbare Dentalwerkstoffe, die eine hohe Durchhärtungstiefe mit guten ästhetischen Eigenschaften kombinieren. Die Werkstoffe sind dadurch gekennzeichnet, dass die zu ihrer Herstellung verwendete Monomermischung einen Brechungsindex n_{D} von 1,50 bis 1,70 aufweist und dass der Brechungsindex der Monomermischung vor der Härtung dem Brechungsindex des Füllstoffs entspricht oder um maximal 0,013 größer ist, nach der Härtung aber um mindestens 0,02 größer als der Brechungsindex des Füllstoffs ist. Die Dentalwerkstoffe weisen vor der Polymerisation eine hohe Transluzenz und damit eine große Durchhärtungstiefe auf. Bei der Polymerisation nimmt die Transluzenz ab. Die Materialien können röntgenopake Füllstoffe wie z.B. röntgenopake Gläser oder Ytterbiumfluorid mit einer Partikelgröße von 0,050 bis 2,0 µm enthalten. Die Materialaien sind als Bulk-Fill-Materialien geeignet, sind aber aufgrund ihrer Fließfähigkeit nicht stopfbar.

Die US 4,629,746 offenbart mikrogefüllte Dentalwerkstoffe, die Seltenerdmetallfluoride wie Ytterbiumtrifluorid mit einer Primärteilchengröße von 5 bis 700 nm, vorzugsweise 50 bis 300 nm als röntgenopake Füllstoffe enthalten. Neben den röntgenopaken Füllstoffen können die Materialien nicht röntgenopake Füller wie gefällte oder pyrogene Kieselsäuren enthalten. Die Werkstoffe sollen eine hohe Röntgenopazität und eine gute Transmission aufweisen.

Die EP 1 234 567 A2 offenbart Präpolymerisate mit definierter Korngrößenverteilung, die nur einen geringen Anteil feinkörniger Partikel mit einer Größe von weniger als 10 µm enthalten. Diese Füllstoffe sollen polymerisierbare Zusammensetzungen mit geringem Polymerisationsschrumpf und guter Polierbarkeit, Oberflächenglätte und Abrasionsbeständigkeit ergeben. Zur Erhöhung der Röntgenopazität können die Präpolymerisate röntgenopake Füllstoffe wie Ytterbiumtrifluorid mit einer Partikelgröße von 300 nm enthalten.

Die WO 2017/149242 A1 offenbart die Herstellung von kolloidalen Suspensionen von Ytterbiumfluorid mit einer Partikelgröße von weniger als 100 nm und deren Verwendung zur Herstellung von Dentalwerkstoffen.

Die US 9,833,388 B2 offenbart Dentalwerkstoffe, die Ytterbiumfluorid mit einer Partikelgröße zwischen 25 und 120 nm enthalten. Diese sollen sich durch eine geringe Zahl von Artefakten bei der Volumentomographie auszeichnen.

Die US 2019/0192386 A1 offenbart lichthärtbare Dentalwerkstoffe, die ein polymerisierbares Monomer, anorganischen Füllstoff mit einer Partikelgröße von 0,07 µm oder mehr, einen organisch-anorganischen Füllstoff mit einer Partikelgröße von 0,5 µm oder mehr und einen Photoinitiator enthalten. Die Werkstoffe sollen sich durch eine große Durchhärtungstiefe und gute Verarbeitbarkeit auszeichnen und nach der Härtung im Aussehen gut mit dem natürlichen Zahn harmonieren.

Neben dem absoluten Schrumpf eines Komposits wird der Schrumpfkraft eine zunehmend größere Bedeutung beigemessen. Bei der radikalischen Polymerisation von dentalen Kompositen kommt es aufgrund des Polymerisationsschrumpfes (ΔV_{P}) der eingesetzten Monomere zu einer Volumenkontraktion, die zu einer sehr nachteiligen Randspaltbildung bei Füllungskompositen führen kann. Bei der Polymerisation von monofunktionellen Methacrylaten führt die Schrumpfung bei der Polymerisation nicht zum Aufbau einer Polymerisationsschrumpfungsspannung (PKS), weil die Verringerung des Volumens durch Fließen der gebildeten Makromoleküle kompensiert werden kann. Im Falle der vernetzenden Polymerisation von multifunktionellen Methacrylaten bildet sich aber schon innerhalb weniger Sekunden ein dreidimensionales Polymernetzwerk, das ein viskoses Fließen verhindert, so dass sich eine erhebliche PKS aufbaut.

Die EP 2 965 741 A1 offenbart die Verwendung von radikalisch polymerisierbaren schwefelhaltigen Monomeren wie 2-(Toluol-4-sulfonylmethyl)acrylsäurelaurylester als Kettenregler zur Verringerung der PKS in Dentalwerkstoffen.

Der Erfindung liegt, die Aufgabe zugrunde, Dentalwerkstoffe zur Verfügung zu stellen, die die oben genannten Nachteile nicht haben und die eine hohe Röntgenopazität aufweisen, so dass sie sich gut von der natürlichen Zahnsubstanz unterscheiden lassen. Außerdem sollen die Werkstoffe eine vereinfachte Herstellung ästhetisch ansprechender Restaurationen ermöglichen und sich besonders als dentale Füllungsmaterialien eignen.

Diese Aufgabe wird erfindungsgemäß durch Dentalwerkstoffe gelöst, die
(a) 5 bis 40 Gew.-% mindestens eines radikalisch polymerisierbaren Monomers,
(b) 1 bis 30 Gew.-% Ytterbiumtrifluorid,
(c) 20 bis 90 Gew.-% mindestens eines anorganischen Füllstoffs,
(d) 5 bis 60 Gew.-% mindestens eines Kompositfüllstoffs und
(e) 0,005 bis 3,0 Gew.-% mindestens eines Initiators für die radikalische Polymerisation, vorzugsweise einen Photoinitiator, enthalten,
jeweils bezogen auf die Masse des Dentalwerkstoffs.

Die Dentalwerkstoffe sind dadurch gekennzeichnet, dass das **radikalisch polymerisierbare Monomer (a)** eine Mischung von
(a-1) 20 bis 80 Gew.-% an Urethandi(meth)acrylaten, die
   - 5 bis 60 Gew.-% Tetramethylxylylendiurethandimethacrylat der Formel V380, 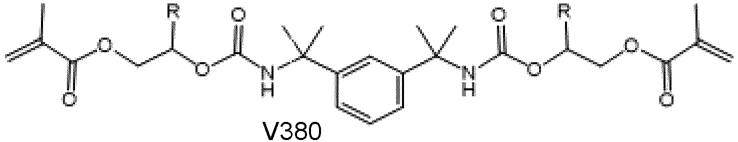 in der die Reste R unabhängig voneinander H oder CH₃ sind,
   - 3 bis 30 Gew.-% mindestens eines difunktionellen Urethans der Formel 1, 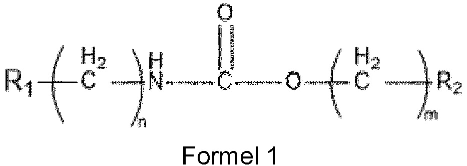 mit
      R¹, R² = unabhängig voneinander jeweils H₂C=C(-R³)-C(=O)-O- oder H₂C=C(-R⁴)-C(=O)-NR⁵-;
      R³ = H oder CH₃;
      R⁴ = H oder CH₃;
      R⁵ = H oder CH₃;
      n, m = unabhängig voneinander jeweils eine ganze Zahl von 1 bis 4, und
   - 10 bis 70 Gew.-% 1,6-Bis-[2-methacryloyloxy-ethoxycarbonylamino]-2,2,4-trimethylhexan enthalten,
(a-2) 10 bis 40 Gew.-% mindestens eines radikalisch polymerisierbaren Bisphenol-A-dimethacrylats,
(a-3) bis zu 40 Gew.-% mindestens eines tricyclischen Dimethacrylats,
(a-4) bis zu 20 Gew.-% sonstiger Monomere, d.h. Monomere, die nicht in eine der Gruppen (a-1) bis (a-3) und (a-5) fallen, und
(a-5) bis zu 8 Gew.-% mindestens eines Kettenreglers, enthält,
jeweils bezogen auf die Gesamtmasse der Komponente (a), wobei die Monomerkomponente (a) einen Brechungsindex von 1,495 bis 1,520 hat.

Das **Ytterbiumtrifluorid (b)** weist eine volumengemittelte Partikelgröße (D50-Wert) von ≤ 25 nm auf.

Der **anorganische Füllstoff (c)** enthält mindestens ein Glaspulver mit einem Brechungsindex im Bereich von 1,49 bis 1,52 und einer volumengemittelten Teilchengröße (D50-Wert) von 0,4 bis 0,9 µm.

Der **Kompositfüllstoff (d)** hat eine durchschnittlichen Teilchengröße von 5 bis 100 µm und weist folgende Zusammensetzung auf:
- 8 bis 50 Gew.-% radikalisch polymerisierbares Monomer, das aus Di(meth)acrylaten, Alkylendimethacrylaten, Urethandimethacrylaten, und Mischungen davon ausgewählt ist,
- 1 bis 20 Gew.-% Ytterbiumtrifluoridpartikel mit einer volumengemittelten Teilchengröße (D50-Wert) von ≤ 25 nm,
- 40 bis 90 Gew.-% weiteren anorganischen Füllstoff und
- 0,01 bis 2 Gew.-% Initiator für die radikalische Polymerisation,
jeweils bezogen auf die Gesamtmasse des Kompositfüllstoffs.

Der Brechungsindex der Monomerkomponente (a) entspricht dem Brechungsindex des Füllstoffs (c) oder ist maximal 0,03 größer und entspricht dem Brechungsindex des Füllstoffs (d) oder ist maximal 0,025 größer.

Die Partikel des Kompositfüllstoffs (d) haben vorzugsweise eine sphärische Form.

Es wurde gefunden, dass durch eine gezielte Auswahl an sich bekannter Stoffe Dentalwerkstoffe hergestellt werden können, die die obigen Anforderungen erfüllen.

Das **Monomer (a)** enthält radikalisch polymerisierbare, difunktionelle Methacrylate und vorzugsweise auch difunktionelle Hybridmonomere. Hybridmonomere sind Monomere, die sowohl (Meth)acrylamid- als auch (Meth)acrylatgruppen enthalten. Unter difunktionellen Monomeren werden Verbindungen mit zwei radikalisch polymerisierbaren Gruppen verstanden. Monomeren mit zwei oder mehr, vorzugsweise 2 bis 4 radikalisch polymerisierbaren Gruppen werden auch als polyfunktionelle Monomere bezeichnet.

Die erfindungsgemäßen Werkstoffe enthalten gemäß einer bevorzugten Ausführungsform keine monofunktionellen Monomere. Unter monofunktionellen Monomeren werden Verbindungen mit einer radikalisch polymerisierbaren Gruppe verstanden. Bevorzugt sind Werkstoffe, die ausschließlich poly- und insbesondere difunktionelle Methacrylate als Komponente (a) enthalten.

Als Komponente (a) wird eine Monomermischung verwendet. Erfindungsgemäß sind Monomere und Monomermischungen bevorzugt, die bei der Polymerisation eine große Änderung des Brechungsindexes zeigen. Die Monomerkomponente (a) hat einen Brechungsindex von 1,495 bis 1,520, bevorzugt von 1,505 bis 1,515. Der Brechungsindex der Monomermischung wird so eingestellt, dass er vor der Härtung dem Brechungsindex des Füllstoffs (c) entspricht oder maximal 0,03 darüber liegt. Vorzugsweise ist der Brechungsindex der Monomermischung um 0,002 bis 0,02, besonders bevorzugt um 0,005 und 0,015 größer als der Brechungsindex des Füllstoffs (c). Der Brechungsindex der Komponente (a) kann durch das Mischen von Monomeren mit unterschiedlichen Brechungsindices eingestellt werden.

Die erfindungsgemäßen Dentalwerkstoffe weisen vor der Polymerisation eine hohe Transluzenz auf, weil die Brechungsindices von Monomer und Füllstoff nur wenig voneinander abweichen. Das zur Polymerisation eingesetzte Licht kann daher tief in die Materialien eindringen, was eine große Durchhärtungstiefe gewährleistet. Bei der Polymerisation nimmt der Brechungsindex der Monomere zu, während der Brechungsindex des oder der Füllstoffe unverändert bleibt. Dadurch vergrößert sich die Differenz zwischen den Brechungsindices von Monomer und Füllstoff, und die Transluzenz nimmt dementsprechend ab. Das ist aus ästhetischen Gründen vorteilhaft, weil tiefer liegende Schichten des Zahns mit einer anderen Färbung besser abgedeckt werden.

Die als Komponente (a) verwendeten Monomere werden vorzugsweise so ausgewählt, dass der Brechungsindexunterschied zwischen dem unpolymerisierten und dem polymerisierten Zustand mindestens 0,015, vorzugsweise mindestens 0,02 beträgt. Gemäß einer besonders bevorzugten Ausführungsform beträgt der Brechungsindexunterschied 0,015 bis 0,04, besonders bevorzugt von 0,021 bis 0,035 und ganz besonders bevorzugt von 0,025 bis 0,030.

Erfindungsgemäß besonders bevorzugte Monomere sind 1,6-Bis-[2-methacryloyloxy-ethoxycarbonylamino]-2,2,4-trimethylhexan (RM3; ein Additionsprodukt aus 2-Hydroxyethylmethacrylat und 2,2,4-Trimethylhexamethylendiisocyanat), N-(2-Methacryl-oyloxyethyl)carbaminsäure-(2-methacryloyloxyethyl)ester (V837; CAS-Nr.: 139096-43-8), Tetramethyl-xylylen-diurethan-dimethacrylat (**V380**), Bisphenol-A-dimethacrylat, 2,2-Bis[4-(2-hydroxy-3-methacryloyloxypropyl)phenyl]propan (BisGMA), ethoxy- oder propoxyliertes Bisphenol-A-Dimethacrylat, wie z.B. das Bisphenol-A-Dimethacrylat 2-[4-(2-Methacryloyloxyethoxyethoxy)phenyl]-2-[4-(2-methacryloyloxy-ethoxy)phenyl]propan) (**SR-348c**; enthält 3 Ethoxygruppen), 2,2-Bis[4-(2-methacryl-oxypropoxy)phenyl]propan, 2-{[(2-(N-Methylacrylamido)-ethoxy)-carbonyl]-amino}-ethylmethacrylat (**V850**, CAS-Nummer: 2004672-68-6), Bis-(3-methacryloyloxy-methyl)tricyclo-[5.2.1.0^{2,6}]decan (**TCP**), 1,10-Decandioldimethacrylat (**D₃MA**) und Mischungen davon.

Die Komponente (a) enthält Tetramethylxylylendiurethandi(meth)acrylat (V380):

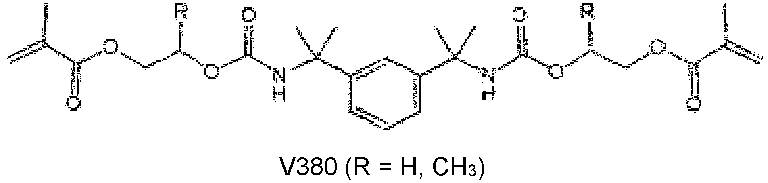

In der gezeigten Formel sind die Reste R unabhängig voneinander H oder CH₃, wo-bei die Reste die gleiche Bedeutung oder unterschiedliche Bedeutungen haben können. Vorzugsweise wird eine Mischung eingesetzt, die Moleküle, in denen beide Reste H sind, Moleküle, in denen beide Reste CH₃ sind, und Moleküle enthält, in denen ein Rest H und der andere Rest CH₃ ist. Eine solche Mischung ist beispielsweise durch Reaktion von 1,3-Bis(1-isocyanato-1-methylethyl)benzol mit Hydroxypropylmethacrylat und 2-Hydroxyethylmethacrylat erhältlich. Ganz besonders bevorzugt ist Tetramethyl-xylylen-diurethan-dimethacrylat (R = CH₃).

Das Urethandimethacrylatmonomer V380 wird in einer Gesamtmenge von 5 bis 60 Gew.-%, bevorzugt von 10 bis 45 Gew.-% und besonders bevorzugt 10 bis 25 Gew.-% bezogen auf die Masse der Monomerkomponente (a) eingesetzt.

Die erfindungsgemäßen Dentalwerkstoffe enthalten mindestens ein Uret-handi(meth)acrylatmonomer und/oder Hybridmonomer der allgemeinen Formel 1: 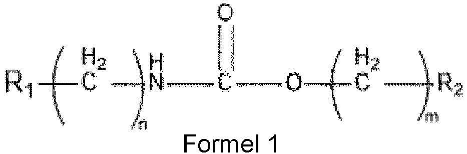 mit
- R¹, R² =: unabhängig voneinander jeweils H₂C=C(-R³)-C(=O)-O- oder H₂C=C(-R⁴)-C(=O)-NR⁵-;
- R³ =: H oder CH₃, vorzugsweise CH₃;
- R⁴ =: H oder CH₃, vorzugsweise H;
- R⁵ =: H oder CH₃, vorzugsweise CH₃;
- n, m =: unabhängig voneinander jeweils eine ganze Zahl von 1 bis 4, vorzugsweise 1 bis 2 und besonders bevorzugt 2.

Monomere der Formel 1 werden im Folgenden auch als difunktionelle Urethane bezeichnet.

Bevorzugt sind difunktionelle Urethane der Formel 1, die einen Brechungsindex von 1,450 bis 1,510, besonders bevorzugt von 1,460 bis 1,505 und ganz besonders bevorzugt von 1,460 bis 1,500 aufweisen.

Besonders bevorzugte difunktionelle Urethane der Formel 1 sind 2-{[(2-(N-Methylacrylamido)-ethoxy)-carbonyl]-amino}-ethylmethacrylat (V850, CAS-Nummer: 2004672-68-6) und insbesondere N-(2-Methacryloyloxyethyl)carbaminsäure-(2-meth-acryloyloxyethyl)ester (V837, CAS-Nr.: 139096-43-8):

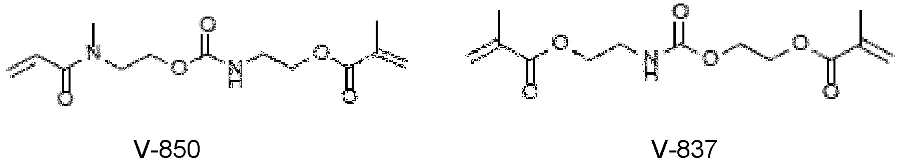

Urethane der Formel 1 zeichnen sich dadurch aus, dass sie bei der Polymerisation eine deutliche Zunahme des Brechungsindexes zeigen. Beispielsweise ändert V850 seinen Brechungsindex von 1,500 vor auf 1,537 nach der Polymerisation und V837 von 1,476 vor auf 1,518 nach der Polymerisation. Urethane der Formel 1 sind damit bestens dazu geeignet, den Brechungsindexwechsel der Monomermischung zu verstärken. V850 zeichnet sich zudem durch eine sehr geringe Toxizität aus (Zytotoxizität: XTT₅₀ = 1085,6 µg/mL (L929 mouse cell line); Ames-Test: negativ (*Salmonella typhimurium* strains TA 1535, TA 1537, TA 98, TA 100 und *Escherichia coli* WP2 uvrA)).

Difunktionelle Urethane nach Formel 1 werden in einer Gesamtmenge von 3 bis 30 Gew.-%, besonders bevorzugt von 5 bis 25 Gew.-% und ganz besonders bevorzugt von 6 bis 20 Gew.-% eingesetzt, bezogen auf die Masse der Monomerkomponente (a).

Neben den bereits genannten Urethandi(meth)acrylaten und difunktionellen Urethanen der Formel 1 enthalten die erfindungsgemäßen Dentalwerkstoffe 10 bis 70 Gew.-%, bevorzugt 15 bis 60 Gew.-% und besonders bevorzugt 20 bis 47 Gew.-% bezogen auf die Masse der Monomerkomponente (a) des Urethandimethacrylats 7,7(9)9-Trimethyl-4,3-dioxo-3,14-dioxa-5,12-diazohexadecan-1,16-diyl-dimethacrylat (RM3).

Die Gesamtmenge an Urethandi(meth)acrylaten und difunktionellen Urethanen der Formel 1 liegt im Bereich von 20 bis 80 Gew.-%, vorzugsweise 30 bis 70 Gew-% und besonders bevorzugt 40 bis 67 Gew.-%, bezogen auf die Masse der Monomerkomponente (a).

Neben den genannten Monomeren enthält die Monomerkomponente (a) ein oder mehrere radikalisch polymerisierbare Bisphenol A-Derivate, beispielsweise 2,2-Bis[4-(2-hydroxy-3-methacryloyloxypropyl)phenyl]propan (BisGMA), vorzugsweise Bisphenol-A-dimethacrylat, besonders bevorzugt ethoxyliertes oder propoxyliertes Bisphenol-A-Dimethacrylat und ganz besonders bevorzugt 2-[4-(2-Methacryloyl-oxyethoxyethoxy)phenyl]-2-[4-(2-methacryloyloxyethoxy)phenyl]propan) (SR-348c, enthält 3 Ethoxygruppen). BisGMA ist ein Additionsprodukt aus Methacrylsäure und Bisphenol-A-diglycidylether. Da kommerziell erhältliches BisGMA häufig mit Bis-Phenol-A verunreinigt ist, sind erfindungsgemäß Werkstoffe bevorzugt, die kein BisGMA enthalten.

Das oder die Bisphenol A-Derivate werden in einer Gesamtenge von 10 bis 40 Gew.- %, bevorzugt von 12 bis 30 Gew.-% und besonders bevorzugt 14 bis 25 Gew.-% eingesetzt, bezogen auf die Masse der Monomerkomponente (a).

Die Komponente (a) kann vorteilhaft weiterhin Methacrylate aus der Gruppe der tricyclischen Dimethacrylate enthalten, insbesondere Tricyclodecandimethanoldimethacrylate und ganz besonders bevorzugt das Tricyclodecandimethanoldimethacrylat TCP (CAS-Nummer: 42594-17-2). TCP verändert seinen Brechungsindex bei der Polymerisation von 1,501 nach 1,531. Tricyclische Dimethacrylate werden in einer Gesamtmenge von bis zu 40 Gew.-%, vorzugsweise 1 bis 40 Gew.-%, besonders bevorzugt von 5 bis 30 Gew.-% und ganz besonders bevorzugt 10 bis 25 Gew.-%, bezogen auf die Masse der Monomerkomponente (a), eingesetzt.

Neben den genannten Monomeren kann die Monomerkomponente (a) vorteilhaft auch einen oder mehrere sogenannte Kettenregler enthalten. Hierbei handelt es sich um Monomere, die das Kettenwachstum während der Polymerisation steuern. Hierdurch wird eine Verringerung der Schrumpfkraft erreicht. Ein erfindungsgemäß besonders bevorzugter Kettenregler ist 2-[(1-Ethoxy-2-methyl-1-oxopropan-2-yl)oxy]acrylsäureethylester. Bevorzugt sind weiterhin die in der EP 2 965 741 A1 offenbarten, radikalisch polymerisierbaren, schwefelhaltigen Monomere, besonders bevorzugt 2-(Toluol-4-sulfonylmethyl)-acrylsäureethylester. Kettenregler werden vorzugsweise in einer Menge von 0 bis 8 Gew.-%, besonders bevorzugt von 0,1 bis 7 Gew.-% und ganz besonders bevorzugt von 0,5 bis 6 Gew.-%, bezogen auf die Masse der Monomerkomponente (a), eingesetzt. Eine geringe Schrumpfkraft wirkt sich vorteilhaft auf die Randdichtigkeit von Füllungen aus.

Schließlich kann die Monomerkomponente (a) ein oder mehrere weitere radikalisch polymerisierbare Monomere enthalten, die in keine der oben genannten Gruppen fallen, beispielsweise zur Einstellung des Brechungsindexes. Bevorzugte weitere Monomere sind (Meth)acrylamide, z.B. N-disubstituierte (Meth)acrylamide, wie N,N-Dimethylacrylamid, sowie Bis(meth)acrylamide, wie N,N'-Diethyl-1,3-bis(acrylamido)-propan, 1,3-Bis(methacrylamido)-propan, 1,4-Bis(acrylamido)-butan und 1,4-Bis-(acryloyl)piperazin. Besonders bevorzugt sind polyfunktionelle und insbesondere difunktionelle Methacrylate, wie Di-, Tri- oder Tetraethylenglycoldimethacrylat, Trimethylolpropantrimethacrylat, Pentaerythrittetramethacrylat, sowie Glycerindimethacrylat und Glycerintrimethacrylat, 1,4-Butandioldimethacrylat, 1,10-Decandioldimethacrylat (D₃MA), 1,12-Dodecandioldimethacrylat und Mischungen davon.

Das Monomer 1,10-Decandioldimethacrylat (D₃MA) ist besonders bevorzugt. Es zeichnet sich durch einen großen Brechungsindexunterschied zwischen Monomer- und Polymerform aus (1,460 zu 1,500). Es hat außerdem einen sehr niedrigen Brechungsindex und eignet sich daher besonders zur Einstellung eines niedrigen Brechungsindexes der Monomerkomponente (a).

Solche weiteren Monomere werden in einer Gesamtmenge von maximal 20 Gew.-%, bevorzugt 2 bis 20 Gew.-% und besonders bevorzugt von 4 bis 10 Gew.-% eingesetzt, bezogen auf die Masse der Monomerkomponente (a).

Die Gesamtmenge an radikalisch polymerisierbaren Monomeren liegt in einem Bereich von 5 bis 40 Gew.-%, bevorzugt 10 bis 35 Gew.-%, besonders bevorzugt 12 bis 30 Gew.-%, bezogen auf die Gesamtmasse des Dentalwerkstoffs.

Erfindungsgemäß besonders bevorzugt sind Dentalwerkstoffe, in denen die Komponente (a) eine Mischung der folgenden Monomere enthält:
(a-1) 30 bis 70 Gew.-% und ganz besonders bevorzugt 40 bis 67 Gew.-% Urethandimethacrylate,
(a-2) 12 bis 30 Gew.-% und ganz besonders bevorzugt 14 bis 25 Gew.-% mindestens eines Bisphenol A-Derivats, vorzugsweise eines ethoxylierten oder propoxylierten Bisphenol-A-Dimethacrylats, ganz besonders bevorzugte SR-348c,
(a-3) bis zu 40 Gew.-%, bevorzugt 5 bis 30 Gew.-% und ganz besonders bevorzugt 10 bis 25 Gew.-% mindestens eines tricyclischen Dimethacrylats, vorzugsweise Tricyclodecandimethanoldimethacrylat (TCP), und
(a-4) bis zu 20 Gew.-%, vorzugsweise 4 bis 20 Gew.-% und besonders bevorzugt 4 bis 10 Gew.-% sonstige Monomere, d.h. Monomere, die nicht in eine der Gruppen (a-1) bis (a-3) und (a-5) fallen, vorzugsweise D₃MA,
(a-5) bis zu 8 Gew.-%,vorzugsweise 0,1 bis 7 Gew.-% und besonders bevorzugt 0,5 bis 6 Gew.-% mindestens eines Kettenreglers,
jeweils bezogen auf die Gesamtmasse der Komponente (a).

Als Komponenten (a-2) bis (a-5) können in allen Fällen einzelne Monomere oder eine Mischung von mehreren Monomeren eingesetzt werden.

Die Monomere (a-1) bis (a-5) werden vorzugsweise aus den oben definierten Stoffen ausgewählt, wobei erfindungsgemäß solche Dentalwerkstoffe besonders bevorzugt sind, in denen die Komponente (a) ausschließlich die genannten Monomere enthält.

Als Komponente (a-1) wird eine Monomermischung eingesetzt, die
- 5 bis 60 Gew.-%, bevorzugt von 10 bis 45 Gew.-% und besonders bevorzugt 10 bis 25 Gew.-% V380,
- von 3 bis 30 Gew.-%, besonders bevorzugt von 5 bis 25 Gew.-% und ganz besonders bevorzugt von 6 bis 20 Gew.-% mindestens eines difunktionellen Urethans der Formel 1,
- 10 bis 70 Gew.-%, bevorzugt 15 bis 60 Gew.-% und besonders bevorzugt 20 bis 47 Gew.-% RM3 enthält,
jeweils bezogen auf die Gesamtmasse der Monomerkomponente (a).

### Röntgenopake Füllstoffe (b)

Die erfindungsgemäßen Werkstoffe enthalten als **Komponente (b)** YbF₃-Partikel mit einer mittleren Primärteilchengröße von ≤ 25 nm, vorzugsweise von 10 bis 24 nm, besonders bevorzugt von 14 bis 22 nm und insbesondere ca. 20 nm, wobei die Teilchen vorzugsweise in nicht aggregierter und nicht agglomerierter Form vorliegen. Partikel mit einer Teilchengröße von ≤ 25 nm werden hierin als nanoskalig bezeichnet.

Wenn nicht anders angegeben, handelt es sich bei allen Partikelgrößen um volumengemittelte Partikelgrößen (D50-Werte, d.h. 50% der Partikel sind kleiner als der angegebene Wert). Die Partikelgrößenbestimmung im Bereich von 0,1 µm bis 1000 µm erfolgt vorzugsweise mittels statischer Lichtstreuung (SLS), beispielsweise mit einem statischen Laserstreuungs-Partikelgrößen-Analysator LA-960 (Horiba, Japan) oder mit einem Microtrac S100 Partikelgrößen-Analysator (Microtrac, USA). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße in einem Bereich von 5 nm bis 0,1 µm erfolgt vorzugsweise durch dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25°C, z.B. mit einem Malvern Zetasizer Nano ZS (Malvern Instruments, Malvern UK).

Es wurde gefunden, dass YbF₃-Partikel mit einer Größe von kleiner 25 nm eine Erhöhung der Röntgenopazität der Werkstoffe ermöglichen, dabei aber nur einen geringen Effekt auf den Brechungsindex der Zusammensetzung haben. Anders als röntgenopake Gläser setzen sie somit nicht die Verwendung von Monomeren mit einem hohen Brechungsindex voraus, um eine gute Transluzenz zu gewährleisten. Durch den Einsatz von nanoskaligen YbF₃-Partikeln kann auf die Verwendung von bariumhaltigem Glas als röntgenopakem Füllstoff verzichtet werden. Vorteilhaft ist weiterhin, dass die nanoskaligen YbF₃-Partikel keine nennenswerte Eintrübung der Pasten bewirken.

Gemäß einer bevorzugten Ausführungsform sind die YbF₃-Partikel oberflächenmodifiziert. Hierzu werden sie vorzugsweise mit einer organischen Verbindung behandelt, die über funktionelle Gruppen verfügt, die an die Oberfläche der YbF₃Partikel binden können. Bevorzugte funktionelle Gruppen sind Phosphat-, Phosphonat-, Carboxyl-, Dithiophosphat-, und Dithiophosphonatgruppen. Die Oberflächenmodifizierungsmittel weisen vorzugsweise außerdem radikalisch polymerisierbare Gruppen auf, die eine Vernetzung mit der organischen Komponente (a) ermöglichen.

Bevorzugte Oberflächenmodifizierungsmittel sind P-7,10,13,16-Tetraoxaheptadec-1-yl-phosphonsäure, P-[6-[2-[2-(2-Hydroxyethoxy)ethoxy]ethoxy]hexyl]phosphonsäure, 2,3-Di-(methacryloyloxy)-propyl-1-phosphonsäure, 2,3-Di-(methacryloyloxy)-propyl-1-bisphosphonsäure und 3-O-Benzyloxy-2-methacryloyloxy-propyl-1-bisphosphonsäure.

Die erfindungsgemäßen Dentalwerkstoffe enthalten 1 bis 30 Gew.-%, bevorzugt 3 bis 20 Gew.-% und besonders bevorzugt 6 bis 12 Gew.-% nanoskalige YbF₃-Partikel, bezogen auf die Masse des Dentalwerkstoffs.

### Anorganischer Füllstoff (c)

Bevorzugte **anorganische Füllstoffe (c)** sind bariumfreie Glaspulver, insbesondere Strontiumglaspulver und/oder zirkonhaltige Glaspulver. Ein ganz besonders bevorzugtes Glas ist das Glas mit der CAS-Nummer 65997-17-3. Die Glaspulver haben eine durchschnittliche Teilchengröße 0,4 bis 0,9 µm.

Der Brechungsindex der Gläser liegt in einem Bereich von 1,49 bis 1,54, bevorzugt 1,49 bis 1,52 und besonders bevorzugt 1,49 bis 1,51. Diese Gläser ergeben überraschender Weise besonders gute Durchhärtungstiefen.

Anorganische Gläser werden vorzugsweise in einer Menge von 20 bis 80 Gew.-%, besonders bevorzugt 25 bis 70 Gew.-% und ganz besonders bevorzugt von 30 bis 60 Gew.-% eingesetzt, bezogen auf die Gesamtmasse des Dentalwerkstoffs.

Weitere bevorzugte anorganische Füllstoffe (c) sind Zirkonsilikate, beispielsweise mit einer Primärteilchengröße von 2 bis 100 nm, bevorzugt 5 bis 60 nm, besonders bevorzugt 10 bis 40 nm und ganz besonders bevorzugt 20 bis 30 nm. Die Primärteilchen sind sphärisch und aggregiert zu Sekundärpartikeln mit einer Größe 0,5 bis 20 µm, bevorzugt 1 bis 10 µm, besonders bevorzugt von 1 bis 7 µm und ganz besonders bevorzugt 2 bis 6 µm. Sie lassen sich entsprechend US 8,617,306 B2 herstellen.

Durch die Zugabe des Zirkonsilikats kann die Polierbarkeit der erfindungsgemäßen Zusammensetzungen verbessert werden. Der Brechungsindex der Zirkonsilikate liegt vorzugsweise in einem Bereich von 1,490 bis 1,510. Zirkonsilikat wird vorzugsweise in einer Menge von 1 bis 30 Gew.-%, besonders bevorzugt 3 bis 25 Gew.-% und ganz besonders bevorzugt 5 bis 20 Gew.-% eingesetzt, bezogen auf die Gesamtmasse des Dentalwerkstoffs.

Außerdem sind als anorganische Füllstoffe ZrO₂-Partikel bevorzugt, vorzugsweise mit einer mittleren Primärteilchengröße von 0,5 bis 50 nm, besonders bevorzugt von 1 bis 20 nm und ganz besonders bevorzugt von 2 bis 10 nm.

Durch die Zugabe von ZrO₂-Partikeln kann die Röntgenopazität der Werkstoffe weiter erhöht werden. Die ZrO₂-Partikel bewirken zudem eine deutliche Erhöhung des Brechungsindexes der Werkstoffe. Um diesen Effekt auszugleichen, werden ZrO₂-Partikel daher vorzugsweise in Kombination mit Monomeren verwendet, die einen geringen Brechungsindex haben. Bevorzugt sind dünnflüssige Methacrylatmonomere wie TCP, Monomere der Formel 1 und insbesondere D₃MA (BI = 1,460).

Gemäß einer bevorzugten Ausführungsform werden die ZrO₂-Partikel in einem dünnflüssigen Monomer suspendiert. In D₃MA lassen sich beispielsweise 30 bis 50 Gew.- % ZrO₂-Partikel suspendieren, ohne dass eine merkliche Trübung des Monomers zu erkennen ist. Der Brechungsindex einer Suspensionen in D₃MA, die 50 Gew.-% ZrO₂-Partikel mit einer mittleren Größe von 8 nm enthält, beträgt beispielsweise 1,524, der Brechungsindex einer 40 Gew.-%igen Suspension von Partikeln mit einer mittleren Größe von 3 nm 1,494. Reines ZrO₂ hat einen Brechungsindex von 2,150.

ZrO₂ wird vorzugsweise in einer Menge von 0,3 bis 5 Gew.-%, besonders bevorzugt 0,4 bis 4 Gew.-% und ganz besonders bevorzugt 0,5 bis 2 Gew.-% eingesetzt, bezogen auf die Gesamtmasse des Werkstoffs.

Die Gesamtmenge an anorganischem Füllstoff (c) beträgt 20 bis 90 Gew.-%, bevorzugt 30 bis 70 Gew.-%, besonders bevorzugt 40 bis 65 Gew.-%, bezogen auf die Gesamtmasse des Dentalwerkstoffs.

Zur Erzielung einer hohen Durchhärtungstiefe des erfindungsgemäßen Dentalwerkstoffs werden die Brechungsindices des Füllers (c) und der Monomerkomponente (a) aufeinander abgestimmt. Dabei wird die Monomerkomponente (a) auf einen Brechungsindex eingestellt, der identisch mit dem Brechungsindex des Füllstoffs (c) oder maximal 0,03 größer ist. Bevorzugt ist der Brechungsindex der Monomerkomponente (a) 0,002 bis 0,02 und besonders bevorzugt 0,05 bis 0,015 größer als der Brechungsindex des Füllstoffs (c).

Die erfindungsgemäßen Werkstoffe können als Füller (c) einen Füllstoff oder eine Füllstoffmischung enthalten. Bei der Verwendung von Füllstoffmischungen sind Werkstoffe bevorzugt, die als Komponente (c) überwiegend, d.h. zu mehr als 50 Gew.-%, besonders bevorzugt zu mehr als 80 Gew.-% bezogen auf die Gesamtmasse der Komponente (c), ganz besonders bevorzugt ausschließlich solche Füllstoffe enthalten, deren Brechungsindex in dem genannten Bereich liegt.

Der Brechungsindex ist eine Stoffkonstante, die von der Wellenlänge des verwendeten Lichts, von der Temperatur, vom Druck und der Reinheit des Stoffes abhängt. Wenn nicht anders angegeben wird hier unter dem Brechungsindex in allen Fällen der bei Raumtemperatur mit Normlicht D65 gemessene Brechungsindex verstanden (n_{D}). Die Bestimmung des Brechungsindex flüssiger Monomere und Monomermischungen kann mit einem handelsüblichen Abbe-Refraktometer erfolgen.

Die Bestimmung des Brechungsindex (BI) von festen Stoffen, wie z.B. von anorganischen Füllstoffen oder Kompositfüllstoffen, erfolgt nach der Immersionsmethode. Die Stoffe werden bei Raumtemperatur in Mischungen von Flüssigkeiten mit unterschiedlichen Brechungsindices dispergiert (sogenannte Immersionsflüssigkeiten). Dabei zeigen sich die Konturen der Feststoffpartikel umso deutlicher, je größer der Brechzahlunterschied zwischen Flüssigkeit und Feststoff ist. Ändert man nun die Brechzahl der Flüssigkeit so, dass sie der des Feststoffs näher kommt, werden die Partikelkonturen schwächer und verschwinden bei Angleichung der Brechindices völlig. Als Immersionsflüssigkeiten eignen sich Flüssigkeiten mit bekanntem Brechungsindex, z.B. Mischungen aus Benzylsalicylat (n_{D}²⁰ = 1,536) und Triacetin (n_{D}²⁰ = 1,431) oder Bromnaphthalin (n_{D}²⁰ = 1,657). Durch die Variation der Mengenverhältnisse dieser Stoffe kann der Brechungsindex der Mischung an den des zu messenden Feststoffs angepasst werden. Bei Übereinstimmung der Brechungsindices wird der Brechungsindex der Immersionsflüssigkeit mit einen Refraktometer bestimmt.

Zur Verbesserung des Verbundes zwischen den Füllstoffpartikeln und der Polymerisationsmatrix sind die Füllstoffe vorzugsweise oberflächenmodifiziert, besonders bevorzugt durch Silanisierung, ganz besonders bevorzugt mit radikalisch polymerisierbaren Silanen, insbesondere mit 3-Methacryloyloxypropyltrimethoxysilan. Zur Oberflächenmodifizierung von nicht-silikatischen Füllstoffen, z.B. von ZrO₂ oder TiO₂, können auch funktionalisierte saure Phosphate, wie z.B. 10-Methacryloyloxydecyldihydrogenphosphat eingesetzt werden.

### Kompositfüller (d)

Die erfindungsgemäßen Werkstoffe enthalten als **Komponente (d)** mindestens einen Kompositfüllstoff. Unter Kompositfüllstoffen werden organische Polymerpartikel verstanden, die ihrerseits mit anorganischen Füllstoffen gefüllt sind. Die Kompositfüllstoffe haben eine durchschnittliche Teilchengröße von 5 bis 100 µm, bevorzugt 15 bis 60 µm und besonders bevorzugt 20 bis 40 µm.

Im Fall der Kompositfüllstoffe wird der Brechungsindex der gehärteten Polymermatrix vorzugsweise so gewählt, dass er mit dem Brechungsindex des darin enthaltenen anorganischen Füllstoffs übereinstimmt oder um maximal ± 0,2, vorzugsweise maximal ±0,1 und besonders bevorzugt maximal ± 0,01 davon abweicht, so dass die Partikel des Kompositfüllers eine hohe Transluzenz aufweisen. Wird mehr als ein anorganischer Füllstoff zur Herstellung des Kompositfüllers verwendet, weist vorzugsweise die überwiegende Menge der anorganischen Füllstoffe, d.h. mehr als 50 Gew.-%, besonders bevorzugt mehr als 80 Gew.-% bezogen auf die Masse der anorganischen Füllstoffe, einen Brechungsindex in dem genannten Bereich auf.

Die Kompositfüllstoffe werden durch Härten von Kompositpasten hergestellt, die ein oder mehrere radikalisch polymerisierbare Monomere und einen oder mehrere anorganische Füllstoffe enthalten.

Zur Herstellung der Kompositfüllstoffe sind die als Komponenten (b) und (c) genannten Füllstoffe und die als Komponente (e) genannten Initiatoren bevorzugt.

Als radikalisch polymerisierbare Monomere zur Herstellung der Kompositfüllstoffe werden Di(meth)acrylate, ganz besonders bevorzugt Glycerindimethacrylat (GDMA, BI = 1,477), Alkylendimethacrylate, wie z.B. 1,10-Decanedioldimethacrylate (D₃MA, BI = 1,460) und Triethylenglykoldimethacrylat (TEGDMA, BI = 1,461), sowie Urethandimethacrylate, wie RM3 und V837, und insbesondere Urethandimethacrylate mit aromatischen Gruppen, besonders bevorzugt V380, und Mischungen davon verwendet.

1,10-Decanedioldimethacrylat zeichnet sich durch einen besonders niedrigen Brechungsindex (BI) aus. Auch das Urethandimethacrylat RM3 zählt mit einem Brechungsindex von 1,485 zu den niedrig brechenden Monomeren. V380 weist mit 1,513 einen deutlich niedrigeren Brechungsindex auf als BisGMA mit 1,552, verfügt aber über dessen gute mechanische Wirkung auf das Komposit.

Bevorzugte Füllstoffe zur Herstellung des Kompositfüllers sind bariumfreie Glaspulver, insbesondere Strontiumgläser und/oder zirkonhaltige Glasfüllstoffe. Besonders bevorzugt sind Strontiumglasfüller, wobei Strontiumglaspulver mit einer Partikelgröße von 0,4 bis 1 µm ganz besonders bevorzugt sind. Besonders bevorzugt sind weiterhin die oben definierten Zirkonsilikate. Ganz besonders bevorzugte anorganische Füllstoffe zur Herstellung der Kompositfüller sind außerdem die oben definierten ZrO₂-Partikel und das als Komponente (b) verwendete nanoskalige Ytterbiumtrifluorid.

Im Fall aggregierter oder agglomerierter Partikel kann die Primärteilchengröße anhand von TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf ein 50 Å dickes Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Die zur Herstellung des Kompositfüllers verwendeten anorganischen Füllstoffe haben vorzugsweise einen Brechungsindex von 1,48 bis 1,55, besonders bevorzugt 1,50 bis 1,53.

Die erfindungsgemäßen Kompositfüller weisen die folgende Zusammensetzung auf:
- 8 bis 50 Gew.-%, vorzugsweise 10 bis 30 Gew.-% radikalisch polymerisierbares Monomer,
- 1 bis 20 Gew.-%, vorzugsweise 2 bis 15 Gew.-% Ytterbiumtrifluoridpartikel mit einer mittleren Teilchengröße von ≤ 25 nm,
- 40 bis 90 Gew.-%, vorzugsweise 60 bis 80 Gew.-% weiterer anorganischer Füllstoffe und
- 0,01 bis 2 Gew.-%, vorzugsweise 0,1 bis 1 Gew.-% Initiator für die radikalische Polymerisation.

Die Prozentangaben beziehen sich auf die Gesamtmasse des Kompositfüllstoffs.

Die Zusammensetzungen können polymerisiert, gemahlen und als Pulver eingesetzt werden. Die Polymerisation erfolgt vorzugsweise thermisch oder photochemisch. Gemahlene Partikel haben in der Regel eine splitterförmige Form. Die gemahlenen Kompositfüller haben vorzugsweise eine mittlere Partikelgröße von 10 bis 50 µm, besonders bevorzugt von 10 bis 40 µm und ganz besonders bevorzugt von 30 bis 40 µm. Sie enthalten vorzugsweise maximal 10 Gew.-%, bezogen auf die Masse des gemahlenen Kompositfüllstoffs, an Partikeln mit einer mittleren Größe von < 10 µm. Bevorzugte Kompositfüller diesen Typs und Verfahren zu ihrer Herstellung werden in EP 1 234 567 A2 beschrieben.

Gemäß einer besonders bevorzugten Ausführungsform haben die Partikel des Kompositfüllstoffs eine sphärische Form, wobei hierunter auch Partikel verstanden werden, die keine perfekte Kugelform aufweisen. Sphärische Partikel können beispielsweise durch die sogenannte Inflightpolymerisation (Aerosol-Polymerisation) hergestellt werden. Hierzu wird das unpolymerisierte Ausgangsmaterial zur Herstellung des Kompositfüllers in Form kleiner Tröpfchen in eine Polymerisationskammer gesprüht und dann durch Bestrahlung mit Licht einer geeigneten Wellenlänge, bevorzugt im blauen Bereich, polymerisiert. Bei Bedarf kann die polymerisierbare Mischung vor dem Versprühen mit einem geeigneten Lösungsmittel verdünnt werden, um die Partikelgröße einzustellen.

Als Initiatoren für die Lichthärtung eignen sich die als Komponente (e) genannten Photoinitiatoren, besonders 4,4'-Dichlorbenzil oder dessen Derivate sowie Campherchinon, vorzugsweise in Kombination mit einem Amin als Beschleuniger, wie zum Beispiel Ethyl-4-(dimethylamino)-benzoat, sowie Dibenzoylgermaniumderivate wie Bis-(4-methoxybenzoyl)diethylgermanium.

Sphärische Kompositfüllstoffe können als anorganische Füllstoffe ebenfalls die oben genannten Stoffe enthalten, wobei hier Strontiumglasfüller, nanoskaliges YbF₃ und/oder besonders die oben definierten Zirkonsilikate bevorzugt sind. Das Strontiumglaspulver hat vorzugsweise eine Partikelgröße im Bereich von 0,4 bis 1 µm, besonders bevorzugt 0,5 bis 0,8 µm.

Der polymerisierte, sphärische Kompositfüller hat eine mittlere Partikelgröße von 5 bis 100 µm, bevorzugt von 10 bis 80 µm, besonders bevorzugt von 20 bis 50 µm.

Erfindungsgemäß wurde überraschend gefunden, dass sphärische Kompositfüllstoffe (d), insbesondere solche, die ihrerseits sphärische Partikel, wie Zirkonsilikat, und/oder nanoskalige Röntgenopaker wie YbF₃ enthalten, die Durchhärtungstiefe und die Biegefestigkeit der Dentalwerkstoffe signifikant verbessern. Außerdem verbessert die Zugabe sphärischer Kompositfüller die Polierbarkeit und Glanzstabilität der Dentalwerkstoffe. Darüber hinaus verbessern diese Füllstoffe die Handhabung und die Standfestigkeit der Pasten.

Die Brechungsindices des Füllers (d) und der Monomerkomponente (a) werden so aufeinander abgestimmt, dass der Brechungsindex der Komponente (a) dem Brechungsindex des Füllstoffs (d) entspricht oder maximal 0,025 größer ist. Vorzugsweise ist der Brechungsindex der Monomerkomponente (a) maximal bis 0,02, besonders bevorzugt maximal bis 0,01 größer als der Brechungsindex des Füllstoffs (d).

Die erfindungsgemäßen Werkstoffe können als Füller (d) einen Füllstoff oder eine Füllstoffmischung enthalten. Bei der Verwendung von Füllstoffmischungen sind Werkstoffe bevorzugt, die als Komponente (d) überwiegend, d.h. zu mehr als 50 Gew.-%, besonders bevorzugt zu mehr als 80 Gew.-%, jeweils bezogen auf die Gesamtmasse der Komponente (d), besonders bevorzugt ausschließlich solche Kompositfüllstoffe enthalten, deren Brechungsindices die genannte Bedingung erfüllen.

Kompositfüller (d) werden in einer Menge von 5 bis 60 Gew.-%, bevorzugt 10 bis 50 Gew.-% und besonders bevorzugt von 15 bis 40 Gew.-% eingesetzt, bezogen auf die Gesamtmasse des Dentalwerkstoffs.

### Initiator für die radikalische Polymerisation (e)

Die erfindungsgemäßen Werkstoffe enthalten als **Komponente (e)** mindestens einen Initiator für die radikalische Polymerisation, vorzugsweise einen Photoinitiator.

Bevorzugte Photoinitiatoren sind Photosensibilisatoren, vor allem α-Diketone, wie 9,10-Phenanthrenchinon, 1-Phenyl-propan-1,2-dion, Diacetyl oder 4,4'-Dichlorbenzil oder deren Derivate, besonders bevorzugt Campherchinon (CC) und dessen Derivate, und Mischungen davon.

Die Photoinitiatoren werden vorzugsweise in Kombination mit Beschleunigern eingesetzt. Als Beschleuniger eignen sich besonders tertiäre Amine, wie z.B. tertiäre aromatische Amine, insbesondere N,N-Dialkyl-aniline, -p-toluidine oder -3,5-xylidine, p-(N,N-Dialkylamino-phenylethanol, -benzoesäurederivate, -benzaldehyd, -phenylessigsäureester und -phenylpropionsäureester. Konkrete Beispiele hierfür sind N,N-Dimethylanilin, N,N-Dimethyl-p-toluidin, N,N,3,5-Tetramethylanilin, N,N-Dimethyl-amino-p-benzaldehyd, p-(Dimethylamino)-benzoesäureethylester oder p-(Dimethyl-amino)-benzonitril. Geeignet sind auch tertiäre aliphatische Amine, wie z.B. Tri-n-butylamin, Dimethylaminoethan-2-ol, Triethanolamin, Dimethylaminoethylmethacrylat, N,N-Dimethylbenzylamin, oder heterocyclische Amine, wie z.B. 1,2,2,6,6-Pentamethylpiperidin, und Aminosäure-Derivate, wie z.B. N-Phenylglycin. Alternativ können aminfreie Beschleuniger verwendet werden, wie z.B. Sulfinsäuren und Sulfinate, Borate, Enolate, Phosphine oder andere Verbindungen, die aktive Wasserstoffatome enthalten, z.B. heterocylische Verbindungen wie Morpholin-Derivate oder 1,3-Dioxolane.

Besonders bevorzugte Photoinitiatoren sind Acyl- oder Bisacylgermanium-Verbindungen, insbesondere die in der EP 1 905 413 A1 offenbarten Monoacyltrialkyl- und Bisacyldialkylgermanium-Verbindungen, wie z.B. Benzoyltrimethylgermanium, Bisbenzoyldiethylgermanium oder Bis(4-methoxybenzoyl)diethylgermanium. Acyl- und Bisacylgermanium-Verbindungen haben den Vorteil, dass sie sich nach der Bestrahlung entfärben (Bleaching Effekt) und so die Transmission der ausgehärteten Werkstoffe nicht beeinträchtigen. Außerdem handelt es sich um monomolekulare Photoinitiatoren, d.h. sie benötigen keinen Beschleuniger, um ihre volle Aktivität zu erreichen.

Weitere besonders bevorzugte Photoinitiatoren sind Acyl- oder Bisacylphosphinoxide, insbesondere die in EP 0 007 505, EP 0 073 413, EP 0 184 095 und EP 0 615 980 beschriebenen Initiatoren. Bevorzugte Beispiele sind die kommerziell zugänglichen Verbindungen 2,4,6-Trimethylbenzoyldiphenylphosphinoxid (Lucirin^{®} TPO, BASF) und Bis(2,4,6-trimethylbenzoyl)phenylphosphinoxid (Irgacure^{®} 819, Ciba). Acyl- und Bisacylphosphinoxide gehören ebenfalls zur Gruppe der monomolekularen Photoinitiatoren und zeichnen sich durch eine geringe Eigenabsorption aus.

Erfindungsgemäße Zusammensetzungen, die einen der genannten Initiatoren enthalten, können beispielsweise durch Bestrahlen mit Blaulicht (Wellenlängenbereich von 400 bis 500 nm) gehärtet werden, vorzugweise durch Bestrahlen mit einer LED-Lampe mit einer Leistung zwischen 1200 mW/cm² und 3050 mW/cm².

Initiatoren werden vorzugsweise in einer Menge von 0,005 bis 3,0 Gew.-%, besonders bevorzugt 0,01 bis 2,0 Gew.-%, besonders bevorzugt 0,1 bis 1 Gew.-% eingesetzt, bezogen auf die Gesamtmasse des Dentalwerkstoffs.

### Weitere Bestandteile

Die erfindungsgemäßen Zusammensetzungen können außerdem weitere **Additive** enthalten, vor allem Rheologiemodifizierungsmittel, Stabilisatoren, wie z.B. Polymerisationsstabilisatoren, Farbmittel, d.h. Farbpigmente und/oder Farbstoffe, antibakterielle Wirkstoffe, fluoridionenabgebende Additive, optische Aufheller, Fluoreszenzmittel, UV-Absorber, Stoffe zur Verbesserung der Bruchzähigkeit und/oder Effektmittel. Die Gesamtmenge an Additiven beträgt vorzugsweise maximal 4 Gew.-%, besonders bevorzugt maximal 3 Gew.-%, bezogen auf die Gesamtmasse des Werkstoffs.

Die erfindungsgemäßen Dentalwerkstoffe enthalten vorzugsweise:
- 10 bis 35 Gew.-%, besonders bevorzugt 12 bis 30 Gew.-% mindestens eines radikalisch polymerisierbaren Monomeren (a),
- 3 bis 20 Gew.-%, besonders bevorzugt 6 bis 12 Gew.- % Ytterbiumtrifluoridpartikel (b),
- 30 bis 70 Gew.-%, besonders bevorzugt 40 bis 65 Gew.-% anorganischen Füllstoff (c),
- 10 bis 50 Gew.-%, besonders bevorzugt 15 bis 40% Kompositfüllstoff (d) und
- 0,01 bis 2,0 Gew.-%, besonders bevorzugt 0,1 bis 1 Gew.-% Initiator für die radikalische Polymerisation (e).

Besonders bevorzugt sind Dentalwerkstoffe, die folgende Zusammensetzung aufweisen:
- 12 bis 30 Gew.-% radikalisch polymerisierbare Monomeren (a),
- 3 bis 10 Gew.-% Ytterbiumtrifluoridpartikel (b),
- 45 bis 65 Gew.-% anorganischen Füllstoff (c),
- 15 bis 40 Gew.-% Kompositfüllstoff (d) und
- 0,01 bis 0,5 Gew.-% Initiator für die radikalische Polymerisation (e).

Die Prozentangaben beziehen sich jeweils auf die Gesamtmasse des Dentalwerkstoffs.

Die für Komponente (b) gemachten Mengenangaben schließen nicht das ggf. in Komponente (d) enthaltene Ytterbiumtrifluorid ein.

Naturgemäß sind solche Werkstoffe bevorzugt, bei denen die Komponenten (a) bis (e) aus den oben definierten bevorzugten und besonders bevorzugten Materialen ausgewählt sind.

Ganz besonders bevorzugt sind jeweils Werkstoffe, bei denen die Monomerkomponente (a) insgesamt 1 bis 25 Gew.-%, bevorzugt 2 bis 20 Gew.-% und ganz besonders bevorzugt 5 bis 12 Gew.-% V850 und/oder V837, 1 bis 60 Gew.-%, bevorzugt 5 bis 30 Gew.-% und ganz besonders bevorzugt 10 bis 25 Gew.-% V380, 1 bis 70 Gew.-%, bevorzugt 2 bis 66 Gew.-% und ganz besonders bevorzugt 5 bis 46 Gew.-% RM3, und 2 bis 40 Gew.-%, bevorzugt 4 bis 30 Gew.-% und ganz besonders bevorzugt 6 bis 25 Gew.-% SR348C enthält. Die Monomerkomponente (a) enthält außerdem vorzugsweise zusätzlich 2 bis 40 Gew.-%, bevorzugt 7 bis 30 Gew.-% und ganz besonders bevorzugt 10 bis 25 Gew.-% TCP, jeweils bezogen auf die Gesamtmasse der Monomerkomponente (a).

Die erfindungsgemäßen Dentalwerkstoffe enthalten vorzugsweise insgesamt 30 bis 95 Gew.-%, besonders bevorzugt 50 bis 90 Gew.-% und ganz besonders bevorzugt 65 bis 85 Gew.-% an Füllstoffen (Komponenten (b), (c) und (d)), bezogen auf die Gesamtmasse des Dentalwerkstoffs.

Die erfindungsgemäßen Dentalwerkstoffe zeichnen sich durch eine hohe Röntgenopazität aus. Dies ermöglicht eine eindeutige Unterscheidung von der natürlichen Zahnsubstanz. Die Röntgenopazität wird nach der ISO-Norm 4049 bestimmt. Dabei wird ein Prüfkörper aus dem polymerisierten Dentalwerkstoff zusammen mit einer Aluminiumtreppe mit einer Stufenhöhe von 1 mm mit einer Röntgenkamera aufgenommen. Man vergleicht den Schwärzungsgrad der Bilder und gibt die Röntgenopazität in % Al an, 100% Röntgenopazität entsprechen dem Schwärzungsgrad von 1 mm Aluminium. Die erfindungsgemäßen Werkstoffe haben vorzugsweise eine Röntgenopazität von 140% bis 350% Al, besonders bevorzugt von 160% bis 250% Al.

Die Röntgenopazität wird vorzugsweise durch die Zugabe von nanoskaligen YbF₃-Partikeln mit einer mittleren Partikelgröße von ≤ 25 nm (Komponente b) erhalten. Besonders bevorzugt sind Dentalwerkstoffe, in denen zusätzlich auch der Kompositfüller (d) nanoskalige YbF₃-Partikel mit einer mittleren Partikelgröße von ≤ 25 nm enthält. Die erfindungsgemäßen Dentalwerkstoffe enthalten vorzugsweise insgesamt, d.h. in den Komponenten (b) und (d), 2 bis 30 Gew.-%, besonders bevorzugt 3 bis 20 Gew.-% und ganz besonders bevorzugt 4 bis 12 Gew.-% nanoskaliges YbF₃, bezogen auf die Gesamtmasse des Werkstoffs.

Die erfindungsgemäßen Dentalwerkstoffe zeichnen sich weiterhin durch eine große Durchhärtungstiefe aus. Die Durchhärtungstiefe wird entsprechend der DIN EN ISO 4049:2018-04 bestimmt und beträgt vorzugsweise 3 mm oder mehr, besonders bevorzugt 3,5 bis 5 mm. Vorteilhaft ist, dass sich diese Durchhärtungstiefen bei den erfindungsgemäßen Werkstoffen mit einer kurzen Belichtungszeit von nur 3 Sekunden (bei 3050 mW/cm²) realisieren lassen.

Ein besonderer Vorteil der erfindungsgemäßen Dentalwerkstoffe sind ihre hervorragenden ästhetischen Eigenschaften. Diese ermöglichen es, eine ästhetisch in jeder Hinsicht überzeugende Dentalrestauration mit nur einem Material herzustellen. Es ist nicht erforderlich, mehrere Materialien miteinander zu kombinieren, um eine ansprechende Restauration herzustellen. Darüber hinaus kann mit nur wenigen Farben der gesamte natürlich vorkommende Farbraum humaner Zähne abgedeckt werden.

Dieser Effekt wird durch ein bestimmtes Verhältnis von Kontrastwert (CR-Wert) zu Transmission erzielt. Die erfindungsgemäßen Dentalwerkstoffe weisen vorzugsweise einen CR-Wert von 60 bis 75, besonders bevorzugt von 62 bis 70 und ganz besonders bevorzugt von 64 bis 68 auf. Die Transmission der eingefärbten Werkstoffe liegt vorzugsweise zwischen 8 und 25%, besonders bevorzugt zwischen 9 und 22% und ganz besonders bevorzugt zwischen 10 und 18%. Alle Angaben beziehen sich auf die gehärteten Materialien.

Unter dem CR-Wert versteht man das Verhältnis der Transmissionsmessungen über einem weißen und einem schwarzen Hintergrund. Der Wert wird auch als Trübheit bezeichnet. Der Kontrastwert CR wird gemäß BS 5612 (Britischer Standard) unter Verwendung eines Spektralkolorimeters (z.B. Minolta CM-3700d) bestimmt. Die Bestimmung des Kontrastwerts besteht aus zwei Einzelmessungen. Das zu analysierende Teststück wird hierfür vor einem schwarzen Keramikkörper mit einer Reflektion von maximal 4 % und entsprechend vor einem weißen Keramikkörper mit einer Reflektion von minimal 86 % angeordnet und diese dann kolorimetrisch analysiert. Bei Verwendung von hochtransparenten Teststücken wird Reflektion/Absorption hauptsächlich durch den keramischen Hintergrund hervorgerufen, wohingegen die Reflektion durch das Teststück hervorgerufen wird, wenn ein opakes Material verwendet wird. Das Verhältnis von reflektiertem Licht vor schwarzem Hintergrund zu reflektiertem Licht vor weißem Hintergrund ist das Maß für den Kontrastwert, wobei vollständige Transmission zu einem Kontrastwert von 0 und vollständige Opazität zu einem Kontrastwert von 100 führt.

Das Zusammenspiel von CR-Wert und Transmission ergibt Werkstoffe mit herausragen ästhetischen Eigenschaften. Eine Transmission im erfindungsgemäßen Bereich erlaubt es dem Umgebungslicht in Werkstoff einzudringen und lässt ihn lebendig erscheinen. Gleichzeitig wird in Werkstoffen mit einem erfindungsgemäßen CR-Wert die in den Werkstoff einstrahlende Farbe der umgebenden Zahnhartsubstanz so gebrochen, dass der Werkstoff eine ähnliche Farbe wie die Zahnhartsubstanz zu haben scheint.

Aufgrund dieser Eigenschaften können die erfindungsgemäßen Werkstoffe mit wenigen Farben den Farbraum der natürlichen Zahnfarben vollständig abdecken, der üblicherweise die 16 Farben der VITA classical A1-D4^{®} Farbskala umfasst. Bei den erfindungsgemäßen Werkstoffen deckt jede Farbe aufgrund des definierten CR-Werts und der definierten Transmission in Kombination ihrer spezifischen Farb- und Helligkeitseinstellung mehrere Farben der üblichen 16 Farben ab. Die Werkstoffe integrieren sich ideal in den natürlichen Zahn, da sie einerseits die Farbe der umgebenden Zahnhartsubstanz aufnehmen und gleichzeitig farbig und opak genug sind, um einen gräulichen Eindruck zu vermeiden.

Die Dentalwerkstoffe eignen sich primär zur intraoralen Anwendung durch den Zahnarzt zur Restauration geschädigter Zähne (therapeutische Anwendung), insbesondere als dentale Zemente, Beschichtungs- oder Verblendmaterialien und ganz besonders als Füllungskomposite und als sogenannte Bulk-Fill-Komposite.

Die erfindungsgemäßen Werkstoffe weisen eine hohe Standfestigkeit und eine geringe Klebrigkeit auf und sind stopfbar. D.h., sie lassen sich ähnlich wie Amalgam verarbeiten und in Zahnkavitäten einbringen und verdichten. Sie eignen sich daher hervorragend als Zahnfüllungsmaterialien, insbesondere für direkte und indirekte Front- und Seitenzahnfüllungen aller Klassen. Diese Eigenschaften werden durch die erfindungsgemäße Wahl von Monomeren, Füllstoffart und Füllstoffmenge erzielt.

Die erfindungsgemäßen Dentalwerkstoffe zeichnen sich durch eine vorteilhafte Kombination von Eigenschaften aus. Die Erfindung ermöglicht es, Werkstoffe mit einem hohen, für Zahnfüllungsmaterialien vorteilhaften Füllstoffgehalt herzustellen, ohne die Durchhärtungstiefe und die ästhetischen Eigenschaften der Materialien zu beeinträchtigen. Aufgrund ihrer optischen Eigenschaften lassen sich die erfindungsgemäßen Werkstoffe daher auch in großen Schichtdicken sehr gut mit Licht härten. Sie eignen sich damit besonders zur Verwendung als Bulk-Fill-Komposite. Unter Bulk-Fill-Kompositen werden dentale Füllungsmaterialien verstanden, die in Schichten von mehr als 3 mm Dicke, vorzugsweise mehr als 4 mm und insbesondere von 4 bis 5 mm Dicke mit Licht gehärtet werden können. Sie erlauben die Herstellung auch großer Zahnfüllungen mit nur 1 bis 2 Schichten.

Die erfindungsgemäßen Werkstoffe können auch extraoral (nicht therapeutisch) eingesetzt werden, beispielsweise bei der Herstellung oder Reparatur von Dentalrestaurationen (nicht therapeutische Anwendung). Sie eignen sich insbesondere als Materialien zur Herstellung von Inlays, Onlays, Kronen oder Brücken.

Die Erfindung wird im Folgenden anhand von Figuren und Ausführungsbeispielen näher erläutert:
Fig. 1 zeigt eine Klasse 2 Kavität in einem humanen Molaren mit stark eingefärbtem Kavitätenboden.
Fig. 2 zeigt den humanen Molaren aus Fig. 1, gefüllt mit dem erfindungsgemäßen Dentalwerkstoff aus Beispiel 5.
Fig. 3 zeigt eine rasterelektronenmikroskopische Aufnahme der sphärischen Partikel aus Beispiel 7.
Fig. 4 zeigt einen gebleichten, humanen Frontzahn mit Klasse 3 mesio-bukkalen und disto-bukkalen Füllungen, die mit dem Dentalwerkstoff aus Beispiel 9 gelegt wurden. Die Füllungen fügen sich natürlich in den Zahn ein und sind praktisch nicht zu erkennen.
Fig. 5 zeigt einen humanen Frontzahn mit Klasse 3 mesio-bukkalen und disto-bukkalen Füllungen, die mit dem Dentalwerkstoff aus Beispiel 13 gelegt wurden. Die Füllungen fügen sich natürlich in den Zahn ein und sind praktisch nicht zu erkennen.

### Ausführungsbeispiele

Mit den in den folgenden Ausführungsbeispielen angegebenen Rezepturen wurden dentale Dentalwerkstoffe hergestellt und wie beschrieben untersucht. Die Komponenten wurden mit einem Magnetrührer, einem Kneter (Firma Linden, Maschinentyp LPM 0.5 SP) bzw. mit einem Zentrifugalmischer (Speedmixer DAC 600.2 der Firma Hausschild) miteinander gemischt.

Zur Bestimmung der Transmission der Werkstoffe wurden gehärtete, runde Prüfkörper (Durchmesser: 20 mm, h = 1 mm) hergestellt und farbmetrisch mit Hilfe eines Spektralphotometers (Spectrophotometer CM-5, Minolta) vermessen. Die Polymerisation erfolgte mit einer LED- Lampe (3 s bei 3050 mW/cm²).

Die Messung von Biegefestigkeit und Durchhärtungstiefe erfolgte nach ISO 4049:2009: Zahnheilkunde - Polymerbasierende Restaurationsmaterialien. Dabei entspricht der angegebene Wert der Durchhärtungstiefe (DHT) dem halben gemessenen Wert. Ab einem gemessenen Wert von DHT/2 ≥ 3,5 mm darf ein Material als Bulk-Fill-fähig bezeichnet werden und eine Durchhärtungstiefe von mindestens 4 mm unter zahnärztlichen Bedingungen gilt als gesichert.

Die Vickershärte wurde mit einem Vickershärtemessgerät der Firma Zwick (ZHV 0.2) bestimmt. Zusätzlich wird der Durchhärtungstiefe [in mm] angegeben, bei der die Vickershärte eines polymerisierten, quer bis zur Mitte abgeschliffenen Prüfkörpers noch 80% der Oberflächenhärte beträgt.

Die Röntgenopazität und der CR-Wert wurden auf die in der Beschreibung beschriebene Weise bestimmt.

In den Beispielen werden die folgenden Materialien verwendet:

| | |
|---|---|
| Beschleuniger | Ethyl-4-(dimethylamino)benzoat (CAS-Nr. 10287-53-3) |
| BisGMA | Bisphenol-A-glycidylmethacrylat (CAS-Nr. 1565-94-2) |
| BHT | Butylhydroxytoluol |
| TCP | Tricyclodecandimethanoldiacrylat (CAS-Nr. 42594-17-2) |
| D₃MA | Decandiol-1,10-dimethacrylat |
| Ge-Photoinitiator | Bis(4-methoxybenzoyl)diethylgermanium (CAS-Nr. 1469766-31-1) |
| Glasfüller | Barium-freies Sr-, Al- und F-haltiges Dentalglas mit 6% Silanisierung, mittlere Korngröße 0,7 µm, Brechungsindex 1,50 (Glas G018-163) |
| Kettelregler | 2-(Toluol-4-sulfonylmethyl)acrylsäureethylester |
| RM3 | 7,7(9)9-Trimethyl-4,3-dioxo-3,14-dioxa-5,12-diazohexadecan-1,16-diyl-dimethacrylat |
| Zirkonsilikat | sphärische Zirkonsilikatpartikel, mittlere Primärpartikelgröße: 20 nm, Sekundärpartikelgröße: 3,44µm, Brechungsindex 1,50 |
| SR-348C | ethoxyliertes Bisphenol-A-dimethacrylat (CAS-Nr 41637-38-1) |
| V380 | Urethandimethacrylat mit aromatischen Gruppen |
| V850 | Methacrylsäure-2-{[2-(*N*-methylacrylam ido)-ethoxycarbonyl]-amino}-ethylester |
| nYbF₃ | nanoskaliges Ytterbiumtrifluorid, mittlere Partikelgröße 14 nm |
| YbF₃ | pulverförmiges Ytterbiumtrifluorid, mittlere Partikelgröße 100 nm |
| ZrO₂ | nicht agglomerierte ZrO₂-Partikel mit einer Primärpartikelgröße von 8 nm |
| V837 | N-(2-Methacryloyloxyethyl)carbaminsäure-(2-methacryloyloxy-ethyl)ester (CAS-Nr. 139096-43-8) |

### Beispiel 1

### Herstellung eines Kompositfüllers (Vergleichsbeispiel)

Auf die in Beispiel 1 der EP 1 234 567 A2 beschriebene Weise wurde ein Kompositmaterial mit der in Tabelle 1 angegebenen Zusammensetzung hergestellt. Das Material wurde thermisch gehärtet, anschließend grob zerkleinert und dann mit einer Kugelmühle auf eine mittlere Korngröße von 25 µm gemahlen. Der Brechungsindex der verwendeten Monomermischung betrug vor der Polymerisation 1,484, nach der Polymerisation 1,509. Der Brechungsindex des Kompositfüllers war 1,506.

**Tabelle 1: Zusammensetzung des Kompositfüllers**

| **Bezeichnung** | **Anteil [Gew.-%]** |
|---|---|
| V 380 | 4,4 |
| RM3 | 4,6 |
| D₃MA | 12,3 |
| Dibenzoylperoxid | 0,69 |
| BHT | 0,01 |
| Glasfüller | 78 |
| Summe | 100 |

### Beispiel 2

### Herstellung eines röntgenopaken Kompositfüllers

Auf die in Beispiel 1 der EP 1 234 567 A2 beschriebene Weise wurde ein Kompositmaterial mit der in Tabelle 2 angegebenen Zusammensetzung hergestellt. Hierzu wurden zunächst die Monomeren miteinander gemischt und dann das Ytterbiumtrifluorid in einen Teil der Monomermischung eingearbeitet. Diese wurde mit den restlichen Monomeren gemischt und danach der Glasfüller homogen in resultierende Mischung eingearbeitet. Das Material wurde thermisch gehärtet, anschließend grob zerkleinert und dann mit einer Kugelmühle auf eine mittlere Korngröße von 25 µm gemahlen. Der Brechungsindex der verwendeten Monomermischung betrug 1,482. Nach der Polymerisation lag er bei 1,514. Der Kompositfüller hatte einen Brechungsindex von 1,506.

**Tabelle 2: Zusammensetzung des röntgenopaken Kompositfüllers**

| **Komponente** | **Anteil [Gew.-%]** |
|---|---|
| V380 | 3,85 |
| RM3 | 3,85 |
| D₃MA | 8,13 |
| nYbF₃ | 5,5 |
| Dibenzoylperoxid | 0,66 |
| BHT | 0,01 |
| Glasfüller | 78 |
| Summe | 100 |

### Beispiel 3

### Dentalwerkstoff auf Basis des Kompositfüllers aus Bsp. 1 (Vergleichsbeispiel)

Zur Herstellung eines Dentalwerkstoffs mit der in Tabelle 3 angegebenen Zusammensetzung wurden zunächst die genannten Monomere für 12 Stunden miteinander verrührt, um alle Komponenten zu lösen. Im Anschluss daran wurden die pulverförmigen Komponenten zugegeben und mit einem Mischer (Speedmixer DAC 600.2 VAC-P der Firma Hausschild) homogen zu einer Paste vermischt. Der Brechungsindex der ungehärteten Monomermischung betrug 1,510.

**Tabelle 3: Zusammensetzung der Kompositpaste**

| **Komponente** | **Anteil [Gew.-%]** |
|---|---|
| BisGMA | 5,45 |
| SR-348C | 2,48 |
| V380 | 3,96 |
| RM3 | 12,00 |
| TCP | 2,64 |
| Campherchinon | 0,05 |
| Beschleuniger | 0,21 |
| Ge-Photoinitiator | 0,05 |
| Additive | 0,06 |
| Kompositfüller aus Bsp. 1 | 17,00 |
| Glasfüller | 56,10 |
| Summe | 100 |

Durchhärtungstiefe (DHT/2), Transmission, Biegefestigkeit, E-Modul und Röntgenopazität wurden wie oben beschrieben gemessen. Die Ergebnisse sind in Tabelle 4 angegeben.

**Tabelle 4: Eigenschaften der gehärteten Dentalwerkstoffe**

| **Messgröße** | **Bsp. 3^{*)}** |
|---|---|
| DHT/2 | 4,1 |
| Transmission (%) | 15,14 |
| Biegefestigkeit (MPa) | 111 |
| Durchhärtungstiefe bei 80% Härte [mm] | 6,5 |
| Röntgenopazität (% Al) | 90 |

| | |
|---|---|
| *) Vergleichsbeispiel | |

### Beispiel 4

### Dentalwerkstoff auf Basis des Kompositfüllers aus Beispiel 2

Zur Herstellung eines Dentalwerkstoffs mit der in Tabelle 5 angegebenen Zusammensetzung wurden zunächst die Monomeren BisGMA, RM3 und Sr-348C unter Rühren homogen vermischt und im Anschluss das YbF₃ in die Mischung eingearbeitet, so dass eine transparente Flüssigkeit erhalten wurde. Der Brechungsindex dieser Mischung betrug 1,509 vor und 1,533 nach der Polymerisation. Die Differenz zwischen diesen Werten beträgt 0,024. Danach wurden die restlichen Monomeren und dann die pulverförmigen Komponenten zugegeben und homogen zu einer Paste vermischt.

Der Werkstoff wurde auf die oben beschriebene Weise analysiert. Die Ergebnisse sind in Tabelle 6 angegeben. Alle Werte übertreffen die Anforderung der Dentalnorm EN-ISO 4049.

**Tabelle 5: Zusammensetzung der röntgenopaken Kompositpaste**

| **Komponente** | **Anteil [Gew.-%]** |
|---|---|
| BisGMA | 2,9 |
| SR-348 | 1,4 |
| RM3 | 9,7 |
| TCP | 2,8 |
| V380 | 2,8 |
| V850 | 1,4 |
| Additive | 0,1 |
| Beschleuniger | 0,2 |
| Ge-Photoinitiator | 0,1 |
| Campherchinon | 0,1 |
| Kompositfüller aus Bsp. 2 | 20,0 |
| Glasfüller | 51,3 |
| nYbF₃ | 7,2 |
| Summe | 100 |

**Tabelle 6: Eigenschaften des gehärteten Dentalwerkstoffs**

| **Messgröße** | **Werkstoff** |
|---|---|
| DHT/2 (mm) | 4,3 |
| Transmission (%) | 17,5 |
| Biegefestigkeit (MPa) | 105 |
| Durchhärtungstiefe bei 80% Härte [mm] | 6,6 |
| Röntgenopazität (% Al) | 160 |
| CR-Wert | 60,8 |

### Beispiel 5

### Einfärben des Dentalwerkstoffs aus Beispiel 4

Die Kompositpaste aus Beispiel 4 wurde durch schrittweise Zugabe des Farbpigments Sicotransrot und intensives Mischen auf die folgenden L,a,b,CR-Werte eingestellt. Anschließend wurde die Paste für 5 min bei 23500 Umdrehungen/min in einem Zentrifugalmischer (SpeedMixer, Fa. Hauschild & Co. KG, Deutschland) und 100 mbar entlüftet.

| **L*** | **a*** | **b*** | **CR** |
|---|---|---|---|
| 81 | 6,63 | 26,28 | 63,35 |

| | | | |
|---|---|---|---|
| L*: Helligkeit, a*: Rot-Wert, b*: Gelb-Wert, CR: Kontrastverhältnis (contrast ratio) | | | |

Die Farben wurden gemäß dem L*a*b*-Farbmodell entsprechend DIN EN ISO 11664-4 bestimmt. Die Farbmessung wurde mit einem handelsüblichen Messgerät (Minolta CM-3700d Farbmessgerät) durchgeführt. Die Durchhärtungstiefe (DHT/2) betrug 3,7 mm.

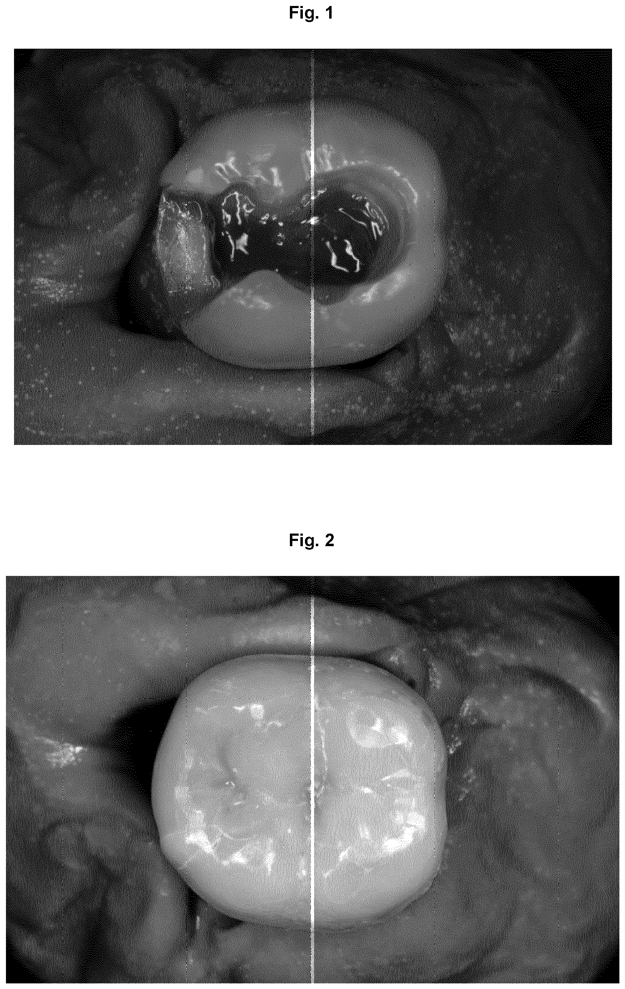

Zur Überprüfung des Abdeckverhaltens wurde ein extrahierter, humaner Seitenzahn entsprechend der Vita Farbe A3.5 aufgebohrt und der Kavitätenboden mit zwei fließfähigen Effektmassen (Empress Direct Color Grau und Empress Direct Color Braun; Firma Ivoclar Vivadent AG) schwarz-gräulich eingefärbt. Fig. 1 zeigt den eingefärbten Kavitätenboden, Fig. 2 zeigt den gleichen Zahn gefüllt mit dem obigen Dentalwerkstoff. Die Verfärbung scheint kaum durch, der Zahn wirkt sehr natürlich. Auf Grund der guten Durchhärtungstiefe lässt sich das Material in einer Schicht in der 4 mm tiefen Kavität aushärten.

### Beispiel 6

### Vergleich von Dentalwerkstoffen mit nanoskaligem und herkömmlichem YbF₃

Auf die in Beispiel 3 beschriebene Weise wurden Werkstoffe mit der in Tabelle 7 angegebenen Zusammensetzung hergestellt (Werkstoffe A und C). Parallel dazu wurde auf die in Beispiel 4 beschriebene Weise ein röntgenopaker Dentalwerkstoff mit der ebenfalls in Tabelle 7 angegebenen Zusammensetzung hergestellt (Werkstoff B). Die Werkstoffe wurden auf die oben beschriebene Weise analysiert. Die Ergebnisse sind in Tabelle 8 angegeben.

**Tabelle 7: Zusammensetzung der Werkstoffe A, B und C**

| **Komponente** | **Werkstoff A^{*)} [Gew.-%]** | **Werkstoff B [Gew.-%]** | **Werkstoff C [Gew.-%]** |
|---|---|---|---|
| BisGMA | 3,88 | 2,90 | 3,88 |
| SR-348C | 1,89 | 1,40 | 1,89 |
| V380 | 3,60 | 2,70 | 3,60 |
| V850 | 1,86 | 1,39 | 1,86 |
| RM3 | 12,61 | 9,45 | 12,61 |
| TCP | 3,66 | 2,74 | 3,66 |
| Additive | 0,08 | 0,08 | 0,08 |
| Campherchinon | 0,05 | 0,05 | 0,05 |
| Beschleuniger | 0,21 | 0,21 | 0,21 |
| Ge-Photoinitiator | 0,05 | 0,05 | 0,05 |
| Kompositfüller aus Bsp. 1 | 20 | 0 | 20 |
| Kompositfüller aus Bsp. 2 | 0 | 20 | 0 |
| Glasfüller | 52,11 | 51,81 | 47,11 |
| YbF₃ (100 nm) | 0 | 0 | 5 |
| nYbF₃ (14 nm) | 0 | 7,22 | 0 |
| Summe | 100 | 100 | 100 |

| | | | |
|---|---|---|---|
| ^{*)} Vergleichsbeispiel | | | |

Die Werkstoffe haben eine ähnliche Zusammensetzung, mit dem Unterschied, dass Werkstoff A kein YbF₃, Werkstoff B nanoskaliges YbF₃ (nYbF₃) und Werkstoff C YbF₃-Pulver mit einer mittleren Teilchengröße von 100 nm enthält. Die Werkstoffe A und B haben eine vergleichbare Durchhärtungstiefe DHT/2 von 4,3 mm bzw. 4,2 mm. Dies zeigt, dass die Zugabe des nanoskaligen YbF₃ die Durchhärtungstiefe nicht signifikant beeinträchtigt. Damit verbleibt ein ausreichend großer Spielraum für eine Einfärbung der Materialien. Farbpigmente und sonstige Farbstoffe können bis zu dem Grenzwert für Bulk-Fill-Materialien von 3,5 mm zugefügt werden. Werkstoff C hingegen hat nur eine DHT/2 von 3,8 mm. Hier ist nur wenig Spielraum für eine Einfärbung vorhanden. Die eintrübende Wirkung von größeren YbF₃-Partikeln wird hier deutlich. Außerdem ist die Differenz zwischen der Transmission vor und nach der Härtung bei Werkstoff C deutlich geringer als bei dem erfindungsgemäß bevorzugten Werkstoff B. Damit ist Werkstoff C als Bulk-Fill Material weniger gut geeignet. Der Vergleich der Werkstoffe A und B zeigt hingegen, dass die Zugabe von nanoskaligem YbF₃ nur einen geringen Einfluss auf die Differenz der Transparenz vor und nach der Härtung hat. Das zeigt, dass nanoskaliges Ytterbiumfluorid bestens geeignet ist, die Röntgenopazität zu erhöhen ohne die optischen Eigenschaften des Werkstoffs maßgeblich zu beeinträchtigen.

**Tabelle 8: Eigenschaften der gehärteten Dentalwerkstoffe**

| **Messgröße** | **Werkstoff A^{*)}** | **Werkstoff B** | **Werkstoff C** |
|---|---|---|---|
| DHT/2 (mm) | 4,3 | 4,2 | 3,8 |
| Transmission (%) vor der Härtung | 40,3 | 36,8 | 29,4 |
| Transmission (%) nach der Härtung | 19,3 | 17,8 | 16,4 |
| Differenz | -21 | -19 | -13 |
| Biegefestigkeit (MPa) | 106 | 106 | 113 |
| Röntgenopazität (% Al) | 90 | 190 | 180 |

### Beispiel 7

### Herstellung Kompositfüllers mit sphärischen Partikeln

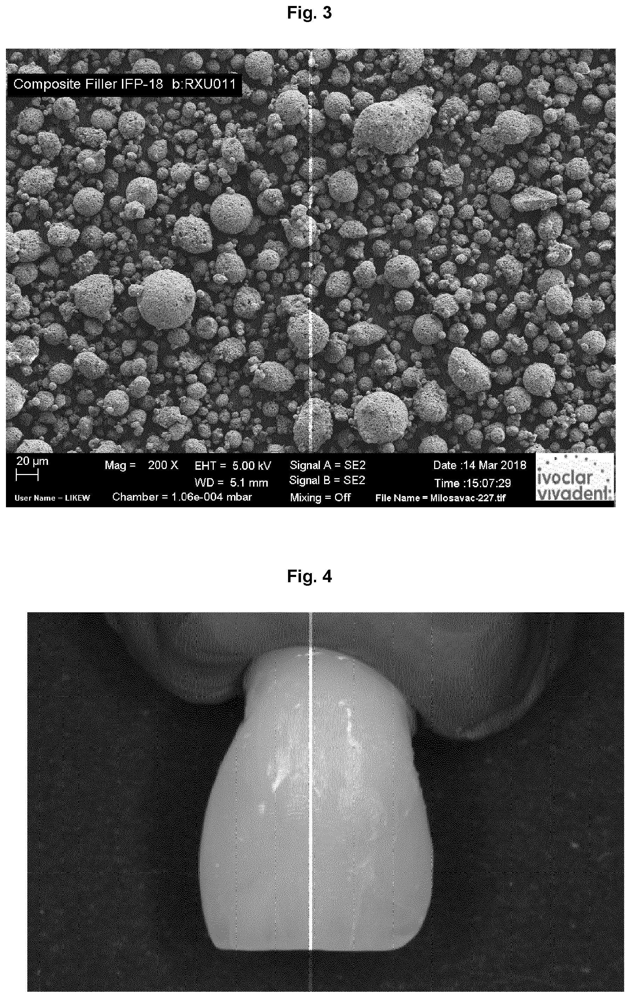

Zur Herstellung eines Kompositfüllers mit der in Tabelle 9 angegebenen Zusammensetzung wurden zunächst die in der Tabelle genannten Monomere miteinander gemischt und dann das Zirkonsilikat in die Monomermischung eingearbeitet. Das Dispergieren erfolgte in einem zylindrischen Glas durch moderates Rühren für 6 bis 24 Stunden. Im Anschluss wurden 0,3 Gew.-% Campherchinon und 0,6 Gew.-% Ethyl-4-(dimethylamino)benzoat zugefügt und weiter gerührt, bis sich die Initiatorkomponenten gelöst hatten. Die Mischung wurde dann mit 20 ml/min in eine Zerstäuberdüse gepumpt, welche mit einem Druck von 2,1 bar unter Stickstoff betrieben wurde. Die fein zerstäubten Tröpfchen wurden mit sechs 100 Watt-LED-Lampen der Wellenlänge 470 nm polymerisiert. Die Größe der gehärteten Partikel wurde mittels Laserbeugung bestimmt (Microtrac X-100 Partikel-Messgerät). Die Partikel hatten eine sphärische Struktur und eine durchschnittliche Partikelgröße von 20 µm. Die Partikelgröße kann durch die Zugabe von Aceton zur Monomermischung vor dem Versprühen (0 bis 25%) kontrolliert werden. Fig. 4 zeigt eine rasterelektronenmikroskopische Aufnahme der sphärischen Partikel. Der Kompositfüller hatte einen Brechungsindex von 1,506.

**Tabelle 9: Zusammensetzung des sphärischen Kompositfüllers**

| **Komponente** | **Anteil [Gew.-%]** |
|---|---|
| V380 | 7,40 |
| RM3 | 8,18 |
| D₃MA | 14,12 |
| Campherchinon | 0,3 |
| Beschleuniger | 0,6 |
| Zirkonsilikat | 69,4 |
| Summe | 100 |

### Beispiel 8

### Herstellung eines röntgenopaken Kompositfüllers mit sphärischen Partikeln

Analog zu der in Beispiel 7 beschriebenen Weise wurde ein sphärischer Kompositfüller mit der in Tabelle 10 beschriebenen Zusammensetzung hergestellt. Der Füller enthält zusätzlich nanoskalige YbF₃-Partikel. Zur Herstellung des Kompositfüllers wurden die in der Tabelle genannten Monomere miteinander gemischt und dann das Ytterbiumtrifluorid und anschließend die weiteren Füllstoffe in die Monomermischung eingearbeitet. Die Monomermischung hatte einen Brechungsindex von 1,478, der Brechungsindex einer Mischung aus 50% Monomermischung und 50% YbF₃ hatte einen Brechungsindex von 1,481. Der Brechungsindex des YbF₃ betrug 1,54.

**Tabelle 10: Zusammensetzung des sphärischen Kompositfüllers**

| **Komponente** | **Anteil [Gew.-%]** |
|---|---|
| V380 | 3,75 |
| RM3 | 4,10 |
| D₃MA | 7,05 |
| nYbF₃ | 15,00 |
| Campherchinon und Beschleuniger | 0,90 |
| Zirkonsilikat | 34,60 |
| Glasfüller | 34,60 |
| Summe | 100 |

### Beispiel 9

### Dentalwerkstoff auf Basis des Kompositfüllers aus Bsp. 7

Zur Herstellung eines Dentalwerkstoffs mit der in Tabelle 11 angegebenen Zusammensetzung wurden zunächst die genannten Monomere unter Rühren homogen vermischt und im Anschluss das YbF₃ in einen Teil der Mischung eingearbeitet, so dass eine weitgehend transparente Flüssigkeit erhalten wurde. Im Anschluss wurden die restlichen Monomere und danach die pulverförmigen Komponenten zugegeben und homogen zu einer Paste vermischt. Der Werkstoff wurde auf die oben beschriebene Weise analysiert. Die Ergebnisse sind in Tabelle 12 angegeben.

Die Paste weist eine sehr gute Durchhärtungstiefe auf. Diese spiegelt sich auch im guten Wert von ca. 7 mm für die Durchhärtungstiefe bei 80% der Vickershärte wieder. Derartige Pasten können problemlos pigmentiert werden, ohne ihre Bulk-Fill-Eigenschaften zu verlieren. Im Vergleich zu Beispiel 4 konnte durch Verwendung des sphärischen Kompositfüllers aus Beispiel 7 an Stelle des gemahlenen Kompositfüllers aus Beispiel 2 eine deutliche Verbesserung der Biegefestigkeit erreicht werden.

**Tabelle 11: Zusammensetzung der röntgenopaken Kompositpaste**

| **Komponente** | **Anteil [Gew.-%]** |
|---|---|
| BisGMA | 2,78 |
| SR-348C | 1,35 |
| RM3 | 9,25 |
| V380 | 2,68 |
| V850 | 4,12 |
| Additive | 0,09 |
| Kettenregler | 1,16 |
| Beschleuniger | 0,21 |
| Ge-Photoinitiator | 0,05 |
| Campherchinon | 0,05 |
| Kompositfüller aus Bsp. 7 | 27,25 |
| Glasfüller | 44,21 |
| nYbF₃ | 6,8 |
| Summe | 100 |

**Tabelle 12: Eigenschaften des gehärteten Dentalwerkstoffs**

| **Messgröße** | **Werkstoff** |
|---|---|
| DHT/2 (mm) | 4,2 |
| Transmission (%) | 17,0 |
| Biegefestigkeit (MPa) | 136 |
| Durchhärtungstiefe bei 80% Vickers-Härte [mm] | 6,9 |

### Beispiel 10

### BisGMA-freier Dentalwerkstoff auf Basis des Kompositfüllers aus Beispiel 8

Zur Herstellung eines BisGMA-freien Dentalwerkstoffs mit der in Tabelle 13 angegebenen Zusammensetzung wurden zunächst die in der Tabelle genannten Monomeren miteinander gemischt und das Ytterbiumtrifluorid dann in die Monomermischung eingearbeitet. Danach wurden die pulverförmigen Komponenten zugegeben und homogen zu einer Paste vermischt. Der Werkstoff wurde auf die oben beschriebene Weise analysiert. Die Ergebnisse sind in Tabelle 14 angegeben.

**Tabelle 13: Zusammensetzung der röntgenopaken Kompositpaste**

| **Komponente** | **Anteil [Gew.-%]** |
|---|---|
| V380 | 4,67 |
| RM3 | 4,87 |
| SR-348C | 3,5 |
| TCP | 4,77 |
| V850 | 1,49 |
| Additive | 2,8 |
| nYbF₃ | 6,6 |
| Beschleuniger | 0,2 |
| Ge-Photoinitiator | 0,05 |
| Campherchinon | 0,05 |
| Kompositfüller aus Bsp. 8 | 20 |
| Glasfüller | 51 |
| Summe | 100 |

**Tabelle 14: Eigenschaften des gehärteten Dentalwerkstoffs**

| **Messgröße** | **Werkstoff** |
|---|---|
| DHT/2 (mm) | 4,8 |
| Transmission (%) vor der Härtung | 45,1 |
| Transmission (%) nach der Härtung | 18,9 |
| Differenz | 26,2 |
| Biegefestigkeit (MPa) | 133 |
| E-Modul (MPa) | 1100 |
| Durchhärtungstiefe bei 80% Vickershärte [mm] | 6,5 |
| Röntgenopazität (% Al) | 182 |
| CR-Wert | 60,17 |

Die Paste weist eine sehr guter Biegefestigkeit und eine hervorragende Durchhärtungstiefe auf. Die große Differenz zwischen der Transmission vor und nach der Härtung ermöglichte es dem Licht tief in die anfänglich sehr transparente Paste einzudringen und den Prüfkörper auch in der Tiefe auszuhärten. Nach der Härtung wies der Werkstoff eine geringere und damit aus ästhetischen Gründen vorteilhafte Transmission auf.

### Beispiel 11

### Einfärbung der Paste aus Beispiel 10 in die Farbe Bleach

Die Kompositpaste aus Beispiel 10 wurde durch schrittweise Zugabe von Weiß-Pigment auf die folgenden L,a,b,CR-Werte eingestellt. Anschließend wurden die Transmission, Durchhärtungstiefe und die Vickershärte gemessen.

| **L*** | **a*** | **b*** | **CR** | **Transmission** | **DHT/2** | **Vickershärte** |
|---|---|---|---|---|---|---|
| 86,84 | 0,59 | 14,65 | 65,52 | 13,08 | 3,5 | 5,5 |

Die erreichte DHT/2 ist normgerecht, und in einer Tiefe von 5,5 mm weist das Material noch 80% der Oberflächenhärte auf.

Fig. 4 zeigt einen gebleichten, humanen Frontzahn mit Klasse 3 mesio-bukkal und disto-bukkalen Füllungen, die mit der eingefärbten Kompositpaste gelegt wurden. Die Füllungen fügen sich natürlich in den Zahn ein. Lediglich aufgrund der vergrößerten Darstellung sind sie überhaupt zu erkennen. Auf Sprechdistanz sind die Füllungen nicht sichtbar.

Füllwerkstoffe der Farbe Bleach eignen sich für sehr helle Zähne wie etwa Milchzähne oder für gebleichte Zähne. Da die Einfärbung auf die Farbe Bleach sehr viel Weisspigment erfordert, um den hellen Eindruck zu erzeugen, führt sie zu einem stärkeren Verlust an Durchhärtungstiefe als andere Farben, die weniger Pigmente erfordern. Aus diesem Grund weisen Werkstoffe mit dieser Einfärbung meist nur eine geringe Durchhärtungstiefe auf. Die obigen Ergebnisse zeigen, dass der erfindungsgemäße Werkstoff selbst mit dieser Farbe eine relative hohe Durchhärtungstiefe hat, die für die Verwendung als Bulk-Fill-Material ausreichend ist. Es ist damit möglich, auch anderen Farben mit ausreichender Durchhärtungstiefe herzustellen.

### Beispiel 12

### Dentalwerkstoff auf Basis des Kompositfüllers aus Beispiel 8 und ZrO₂

Die Herstellung des Dentalwerkstoffs mit der in Tabelle 15 angegebenen Zusammensetzung erfolgte analog zu den zuvor beschriebenen Beispielen. Zusätzlich enthielt die Monomermischung das Monomer D₃MA. Das ZrO₂ wurde in dem D₃MA suspendiert und diese Suspension dann mit den übrigen Bestanteilen gemischt. Die Paste wurde für 3 s bei 3050 mW/cm² polymerisiert und dann auf die in Beispiel 3 beschriebene Weise analysiert. Die Ergebnisse sind in Tabelle 16 angegeben.

Die Kompositpaste weist eine gute Durchhärtungstiefe auf. Im Vergleich zu Beispiel 7 ist die Transmission geringer. Zudem konnte durch den erhöhten Gehalt an YbF₃ und die Zugabe von ZrO₂ der CR-Wert erhöht werden.

**Tabelle 15: Zusammensetzung des Dentalwerkstoffs**

| **Komponente** | **Anteil [Gew.-%]** |
|---|---|
| V380 | 3,66 |
| RM3 | 4,16 |
| SR-348C | 2,85 |
| TCP | 3,45 |
| V850 | 3,59 |
| D₃MA | 0,87 |
| ZrO₂ | 0,58 |
| Additive | 0,08 |
| nYbF₃ | 9,39 |
| Beschleuniger | 0,16 |
| Ge-Photoinitiator | 0,04 |
| Campherchinon | 0,04 |
| Kompositfüller aus Bsp. 8 | 30 |
| Glasfüller | 41,13 |
| Summe | 100 |

**Tabelle 16: Eigenschaften des gehärteten Dentalwerkstoffs**

| **Messgröße** | **Werkstoff** |
|---|---|
| DHT/2 (mm) | 4,1 |
| Transmission (%) | 38,7 |
| vor der Härtung | |
| Transmission (%) | 15,9 |
| nach der Härtung | |
| Differenz | 22,8 |
| Biegefestigkeit (MPa) | 136 |
| Durchhärtungstiefe bei 80% Härte | 6,5 |
| Röntgenopazität (% AI) | 183 |
| CR-Wert (24h nach Polymerisation) | 62,4 |

### Beispiel 13

### Einfärbung der Paste aus Beispiel 12 in eine für dunkle Zähne geeignete Farbe

Die Kompositpaste aus Beispiel 12 wurde durch schrittweise Zugabe der Pigmente Sicotransrot und Xerogel gelb auf die folgenden L,a,b,CR-Werte eingestellt. Anschließend wurden die Transmission, Durchhärtungstiefe und die Vickershärte gemessen.

| **L*** | **a*** | **b*** | **CR** | **Transmission** | **DHT/2** | **Vickershärte** |
|---|---|---|---|---|---|---|
| 78.71 | 9.33 | 31.0 | 65.0 | 13,08 | 3,5 | 5,2 |

Die erreichte DHT/2 ist normgerecht, und in einer Tiefe von 5,5 mm besitzt das Material noch 80% der Oberflächenhärte.

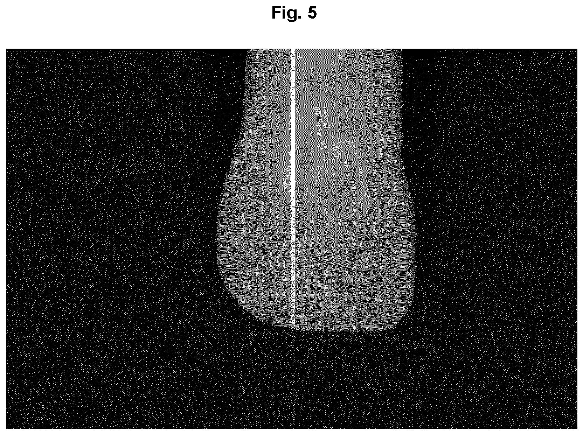

Fig. 5 zeigt einen humanen Frontzahn, der üblicher Weise mit einer Füllung der Farbe A3.5 (Vita-Farbsystem) restauriert würde, mit Klasse 3 mesio-bukkal und disto-bukkalen Füllungen, die mit der eingefärbten Kompositpaste gelegt wurden. Die Füllungen fügen sich natürlich in den Zahn ein. Lediglich aufgrund der vergrößerten Darstellung sind sie überhaupt zu erkennen. Auf Sprechdistanz sind die Füllungen nicht sichtbar.

Dentalwerkstoffe für dunkle Zähne benötigen eine relativ große Menge an Farbpigmenten zur Einstellung der Farbe. Aus diesem Grund solche Werkstoffe meist nur eine geringe Durchhärtungstiefe auf. Die obigen Ergebnisse zeigen, dass der erfindungsgemäße Werkstoff eine relative hohe Durchhärtungstiefe hat, die für die Verwendung als Bulk-Fill-Material ausreichend ist. Es ist damit möglich, auch andere Farben mit ausreichender Durchhärtungstiefe herzustellen.

### Beispiel 14

### Dentalwerkstoff auf Basis des Kompositfüllers aus Beispiel 8 und Zirkonsilikat

Analog zu Beispiel 12 wurde ein Dentalwerkstoff hergestellt, der als Füllstoff zusätzlich Zirkonsilikat und einen höheren Anteil an ZrO₂ enthielt. Die Zusammensetzung ist in Tabelle 17 angegeben. Die Paste wurde für 3 s bei 3050 mW/cm² polymerisiert und dann auf die in Beispiel 3 beschriebene Weise analysiert. Die Ergebnisse sind in Tabelle 18 angegeben. Die Messergebnisse weisen das Komposit als eine bulkfähige Paste mit guter Röntgenopazität aus.

**Tabelle 17: Zusammensetzung des Dentalwerkstoffs**

| **Komponente** | **Anteil [Gew.-%]** |
|---|---|
| V-380 | 3,71 |
| RM3 | 4,05 |
| SR 348C | 2,50 |
| TCP | 3,2 |
| V850 | 3,12 |
| D₃MA | 1,44 |
| ZrO₂ | 0,96 |
| Additive | 0,08 |
| nYbF₃ | 7,2 |
| Beschleuniger | 0,16 |
| Ge-Photoinitiator | 0,04 |
| Campherchinon | 0,04 |
| Kompositfüller aus Bsp. 8 | 30,00 |
| Glasfüller | 33,50 |
| Zirkonsilikat | 10,00 |
| Summe | 100 |

**Tabelle 18: Eigenschaften des gehärteten Dentalwerkstoffs**

| **Messgröße** | **Werkstoff** |
|---|---|
| DHT/2 (mm) | 4,5 |
| Transmission (%) | 37,2 |
| Transmission (%) | 15,2 |
| nach der Härtung | |
| Differenz | 22 |
| Biegefestigkeit (MPa) | 120 |
| Durchhärtungstiefe bei 80% Härte | 6,8 |
| Röntgenopazität (% AI) | 200 |
| CR-Wert (24h nach Polymerisation) | 62 |

### Beispiel 15

### Werkstoffe mit YbF₃ unterschiedlicher Partikelgröße

Es wurden drei Pasten mit der in Beispiel 14 angegebenen Zusammensetzung hergestellt. Dabei wurde das in Beispiel 14 verwendete YbF₃ jeweils durch YbF₃ mit einer anderen Partikelgröße ersetzt: Paste A: 20 nm, Paste B: 40 nm und Paste C: 60 nm. Mit Paste A ließ sich eine Durchhärtungstiefe DHT/2 von 4,4 mm erzielen. Die gröberen Partikel ergaben eine Durchhärtungstiefe von nur 3,8 mm.

### Beispiel 16

### BisGMA-freier Dentalwerkstoff auf Basis des Kompositfüllers aus Beispiel 8 und ZrO₂

Zur Herstellung eines BisGMA-freien Dentalwerkstoffs mit der in Tabelle 19 angegebenen Zusammensetzung wurden zunächst die in der Tabelle genannten Monomeren miteinander gemischt und das Ytterbiumtrifluorid dann in die Monomermischung eingearbeitet. Danach wurden die pulverförmigen Komponenten zugegeben und homogen zu einer Paste vermischt. Der Werkstoff wurde auf die oben beschriebene Weise analysiert. Die Ergebnisse sind in Tabelle 20 angegeben.

Die Paste zeigt eine sehr gute Abnahme der Transmission bei der Polymerisation und einen hohen Wert für die Durchhärtungstiefe bei 80% der Härte. Sie eignet sich daher hervorragend als Bulkfillmaterial.

**Tabelle 19: Zusammensetzung des Dentalwerkstoffs**

| **Komponente** | **Anteil [Gew.-%]** |
|---|---|
| V-380 | 3,5 |
| RM3 | 4,2 |
| SR 348C | 4,1 |
| TCP | 3,27 |
| V837 | 1,8 |
| D₃MA | 1,5 |
| ZrO₂ | 1 |
| Additive | 0,07 |
| nYbF₃ | 7,5 |
| Beschleuniger | 0,04 |
| Ge-Photoinitiator | 0,01 |
| Campherchinon | 0,01 |
| Kompositfüller aus Bsp. 8 | 25 |
| Glasfüller | 48 |
| Summe | 100 |

**Tabelle 20: Eigenschaften des gehärteten Dentalwerkstoffs**

| **Messgröße** | **Werkstoff** |
|---|---|
| DHT/2 (mm) | 4,3 |
| Transmission (%) | 37,7 |
| vor der Härtung | |
| Transmission (%) | 14,7 |
| nach der Härtung | |
| Differenz | 23 |
| Biegefestigkeit (MPa) | 122 |
| Durchhärtungstiefe bei 80% Härte | 8,0 |
| Röntgenopazität (% AI) | 200 |
| CR-Wert (24h nach Polymerisation) | 61 |

## Patentansprüche

1. Dentalwerkstoff, der
(a) 5 bis 40 Gew.-% mindestens eines radikalisch polymerisierbaren Monomers,
(b) 1 bis 30 Gew.-% Ytterbiumtrifluorid,
(c) 20 bis 90 Gew.-% mindestens eines anorganischen Füllstoffs,
(d) 5 bis 60 Gew.-% mindestens eines Kompositfüllstoffs und
(e) 0,005 bis 3,0 Gew.-% mindestens eines Initiators für die radikalische Polymerisation, vorzugsweise einen Photoinitiator, enthält,
jeweils bezogen auf die Masse des Dentalwerkstoffs, **dadurch gekennzeichnet, dass**
das radikalisch polymerisierbare **Monomer (a)** eine Mischung von
(a-1) 20 bis 80 Gew.-% an Urethandi(meth)acrylaten, die
- 5 bis 60 Gew.-% Tetramethylxylylendiurethandimethacrylat der Formel V380, 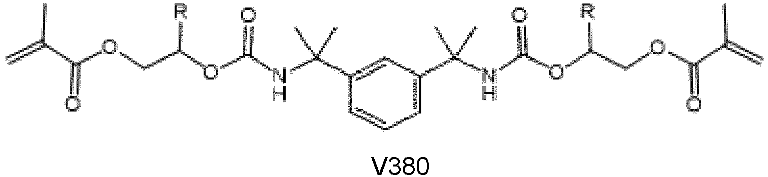 in der die Reste R unabhängig voneinander H oder CH₃ sind,
- 3 bis 30 Gew.-% mindestens eines difunktionellen Urethans der Formel 1, 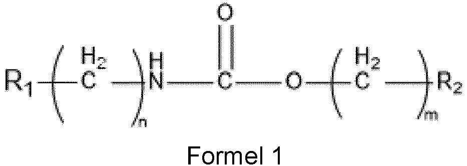 mit
R¹, R² = unabhängig voneinander jeweils H₂C=C(-R³)-C(=O)-O- oder H₂C=C(-R⁴)-C(=O)-NR⁵-;
R³ = H oder CH₃;
R⁴ = H oder CH₃;
R⁵ = H oder CH₃;
n, m = unabhängig voneinander jeweils eine ganze Zahl von 1 bis 4, und
- 10 bis 70 Gew.-% 1,6-Bis-[2-methacryloyloxy-ethoxycarbonyl-amino]-2,2,4-trimethylhexan enthalten,
(a-2) 10 bis 40 Gew.-% mindestens eines radikalisch polymerisierbaren Bisphenol-A-dimethacrylats,
(a-3) bis zu 40 Gew.-% mindestens eines tricyclischen Dimethacrylats,
(a-4) bis zu 20 Gew.-% sonstiger Monomere, d.h. Monomere, die nicht in eine der Gruppen (a-1) bis (a-3) und (a-5) fallen, und
(a-5) bis zu 8 Gew.-% mindestens eines Kettenreglers, enthält,
jeweils bezogen auf die Gesamtmasse der Komponente (a), wobei die Monomerkomponente (a) einen Brechungsindex von 1,495 bis 1,520 hat,
das **Ytterbiumtrifluorid (b)** eine volumengemittelte Partikelgröße (D50-Wert) von ≤ 25 nm aufweist,
der anorganische **Füllstoff (c)** Glaspulver mit einem Brechungsindex im Bereich von 1,49 bis 1,52 und einer volumengemittelten Teilchengröße (D50-Wert) von 0,4 bis 0,9 µm enthält und
der **Kompositfüllstoff (d)** eine durchschnittliche Teilchengröße von 5 bis 100 µm hat und folgende Zusammensetzung aufweist:
- 8 bis 50 Gew.-% radikalisch polymerisierbares Monomer, das aus Di(meth)acrylaten, Alkylendimethacrylaten, Urethandimethacrylaten, und Mischungen davon ausgewählt ist,
- 1 bis 20 Gew.-% Ytterbiumtrifluoridpartikel mit einer volumengemittelten Teilchengröße (D50-Wert) von ≤ 25 nm,
- 40 bis 90 Gew.-% weiteren anorganischen Füllstoff und
- 0,01 bis 2 Gew.-% Initiator für die radikalische Polymerisation,
jeweils bezogen auf die Gesamtmasse des Kompositfüllstoffs,
wobei die Partikelgröße in allen Fällen auf die in der Beschreibung beschriebene Weise gemessen wird, und
wobei der Brechungsindex der Monomerkomponente (a) dem Brechungsindex des Füllstoffs (c) entspricht oder maximal 0,03 größer ist und der Brechungsindex der Monomerkomponente (a) dem Brechungsindex des Füllstoffs (d) entspricht oder maximal 0,025 größer ist.

2. Dentalwerkstoff nach Anspruch 1, der
(a) 10 bis 35 Gew.-% mindestens eines radikalisch polymerisierbaren Monomers,
(b) 3 bis 20 Gew.-% Ytterbiumtrifluorid mit einer volumengemittelten Partikelgröße (D50-Wert) von 10 bis 24 nm,
(c) 30 bis 70 Gew.-% mindestens eines anorganischen Füllstoffs,
(d) 10 bis 50 Gew.-% mindestens eines Kompositfüllstoffs und
(e) 0,01 bis 2,0 Gew.-% mindestens eines Initiators für die radikalische Polymerisation, vorzugsweise einen Photoinitiator, enthält,
jeweils bezogen auf die Masse des Dentalwerkstoffs,
wobei das radikalisch polymerisierbare **Monomer (a)** eine Mischung von
(a-1) 20 bis 80 Gew.-% an Urethandimethacrylaten, die
- 5 bis 60 Gew.-% Tetramethylxylylendiurethandimethacrylat der Formel V380,
- 3 bis 30 Gew.-% mindestens eines difunktionellen Urethans der Formel 1, und
- 10 bis 70 Gew.-% 1,6-Bis-[2-methacryloyloxy-ethoxycarbonyl-amino]-2,2,4-trimethylhexan enthalten,
(a-2) 10 bis 40 Gew.-% mindestens eines von Bisphenol-A-dimethacrylat, 2,2-Bis[4-(2-hydroxy-3-methacryloyloxypropyl)phenyl]propan, 2,2-Bis[4-(2-methacryloxypropoxy)phenyl]propan und 2-[4-(2-Methacryloyloxy-ethoxyethoxy)phenyl]-2-[4-(2-methacryloyloxyethoxy)phenyl]propan),
(a-3) bis zu 40 Gew.-% Bis-(3-methacryloyloxymethyl)tricyclo-[5.2.1.0^{2,6}]-decan, und
(a-4) bis zu 20 Gew.-% sonstiger Monomere, d.h. Monomere, die nicht in eine der Gruppen (a-1) bis (a-3) und (a-5) fallen, und
(a-5) bis zu 8 Gew.-% mindestens eines Kettenreglers, enthält,
jeweils bezogen auf die Gesamtmasse der Komponente (a),
der anorganischen **Füllstoff (c)** bariumfreies Strontiumglaspulver mit einem Brechungsindex im Bereich von 1,49 bis 1,51 und einer volumengemittelten Teilchengröße (D50-Wert) von 0,4 bis 0,9 µm enthält und
der **Kompositfüllstoff (d)** eine durchschnittliche Teilchengröße von 5 bis 100 µm hat und folgende Zusammensetzung aufweist:
- 8 bis 50 Gew.-% radikalisch polymerisierbares Monomer, das aus Glycerindimethacrylat, 1,10-Decanedioldimethacrylat, Triethylenglycoldimethacrylat, 1,6-Bis-[2-methacryloyloxy-ethoxycarbonylamino]-2,2,4-tri-methylhexan, N-(2-Methacryloyloxyethyl)carbaminsäure-(2-methacryl-oyloxyethyl)ester, Tetramethylxylylendiurethandi(meth)acrylat und Mischungen davon ausgewählt ist,
- 1 bis 20 Gew.-% Ytterbiumtrifluoridpartikel mit einer volumengemittelten Teilchengröße (D50-Wert) von 10 bis 24 nm,
- 40 bis 90 Gew.-% bariumfreies Strontiumglaspulver, Zirkonsilikat und/oder ZrO₂-Partikel und
- 0,01 bis 2 Gew.-% Initiator für die radikalische Polymerisation,
jeweils bezogen auf die Gesamtmasse des Kompositfüllstoffs.

3. Dentalwerkstoff nach Anspruch 1 oder 2, bei dem das radikalisch polymerisierbare **Monomer (a-1)** aus 1,6-Bis-[2-methacryloyloxy-ethoxycarbonyl-amino]-2,2,4-trimethylhexan, N-(2-Methacryloyloxyethyl)carbaminsäure-(2-methacryloyloxyethyl)ester, Tetramethylxylylendiurethandimethacrylat der Formel V380 und 2-{[(2-(N-Methylacrylamido)-ethoxy)-carbonyl]-amino}-ethylmethacrylat ausgewählt ist, das **Monomer (a-2)** aus Bisphenol-A-dimethacrylat, 2,2-Bis[4-(2-hydroxy-3-methacryloyloxypropyl)phenyl]propan, ethoxy- oder propoxyliertem Bisphenol-A-Dimethacrylat, 2-[4-(2-Methacryloyl-oxyethoxyethoxy)phenyl]-2-[4-(2-methacryloyloxyethoxy)phenyl]propan) und 2,2-Bis[4-(2-methacryloxypropoxy)phenyl]propan ausgewählt ist, das **Monomer (a-3)** Bis-(3-methacryloyloxymethyl)tricyclo-[5.2.1.0^{2,6}]decan ist und das **Monomer (a-4)** 1,10-Decandioldimethacrylat ist.

4. Dentalwerkstoff nach einem der Ansprüche 1 bis 3, der als radikalisch polymerisierbares Monomer (a) eine Mischung von
(a-1) 20 bis 80 Gew.-%, bevorzugt 30 bis 70 Gew.-% und besonders bevorzugt 40 bis 67 Gew.-% der Monomermischung (a-1),
(a-2) 10 bis 40 Gew.-%, bevorzugt 12 bis 30 Gew.-% und besonders bevorzugt 14 bis 25 Gew.-% mindestens eines von Bisphenol-A-dimethacrylat, 2,2-Bis[4-(2-hydroxy-3-methacryloyloxypropyl)phenyl]propan und/ 2-[4-(2-Methacryloyloxyethoxyethoxy)phenyl]-2-[4-(2-methacryloyl-oxyethoxy)phenyl]propan),
(a-3) ggf. bis zu 40 Gew.-%, bevorzugt 5 bis 30 Gew.-% und besonders bevorzugt 10 bis 25 Gew.-% Tricyclodecandimethanoldimethacrylat, und
(a-4) ggf. bis zu 20 Gew.-%, vorzugsweise 4 bis 20 Gew.-% und besonders bevorzugt 4 bis 10 Gew.-% sonstiger Monomere, d.h. Monomere, die nicht in eine der Gruppen (a-1) bis (a-3) und (a-5) fallen, vorzugsweise 1,10-Decandioldimethacrylat,
(a-5) ggf. bis zu 8 Gew.-%, vorzugsweise 0,1 bis 7 Gew.-% und besonders bevorzugt 0,5 bis 6 Gew.-% mindestens eines Kettenreglers, enthält,
jeweils bezogen auf die Gesamtmasse der Komponente (a).

5. Dentalwerkstoff nach einem der Ansprüche 1 bis 4, bei dem die Variablen der allgemeinen Formel 1 die folgenden Bedeutungen haben:
R¹, R² = unabhängig voneinander jeweils H₂C=C(-R³)-C(=O)-O- oder H₂C=C(-R⁴)-C(=O)-NR⁵-;
R³ = CH₃;
R⁴ = H;
R⁵ = CH₃;
n, m = unabhängig voneinander jeweils eine ganze Zahl von 1 bis 2 und bevorzugt 2.

6. Dentalwerkstoff nach Anspruch 5, der als Komponente (a-1) eine Monomermischung enthält, die
- 10 bis 45 Gew.-% und bevorzugt 10 bis 25 Gew.-% Tetramethylxylylendiurethandimethacrylat der Formel V380,
- von 3 bis 25 Gew.-% und bevorzugt von 6 bis 20 Gew.-% mindestens eines difunktionellen Urethans der Formel 1,
- 15 bis 60 Gew.-% und bevorzugt 20 bis 47 Gew.-% 1,6-Bis-[2-methacryl-oyloxyethoxycarbonylamino]-2,2,4-trimethylhexan enthält,
jeweils bezogen auf die Gesamtmasse der Monomerkomponente (a).

7. Dentalwerkstoff nach einem der Ansprüche 1 bis 6, der als röntgenopaken Füllstoff (b) Ytterbiumtrifluorid mit einer volumengemittelten Partikelgröße (D50-Wert) von 10 bis 24 nm enthält.

8. Dentalwerkstoff nach einem der Ansprüche 1 bis 7, wobei der anorganischen Füllstoff (c) zusätzlich
- ein oder mehrere Zirkonsilikate mit einer volumengemittelten Primärteilchengröße (D50-Wert) von 2 bis 100 nm, besonders bevorzugt 5 bis 60 nm und ganz besonders bevorzugt 10 bis 40 nm und/oder
- ZrO₂-Partikel mit einer volumengemittelten Primärteilchengröße (D50-Wert) von 0,5 bis 50 nm, besonders bevorzugt von 1 bis 20 nm und ganz besonders bevorzugt von 2 bis 10 nm enthält.

9. Dentalwerkstoff nach einem der Ansprüche 1 bis 8, bei dem der Brechungsindex der Monomerkomponente (a) dem Brechungsindex des Füllstoffs (c) entspricht oder maximal 0,002 bis 0,02 und bevorzugt 0,005 bis 0,015 größer ist und bei dem der Brechungsindex der Monomerkomponente (a) dem Brechungsindex des Füllstoffs (d) entspricht oder maximal 0,02 und bevorzugt maximal 0,01 größer ist.

10. Dentalwerkstoff nach einem der Ansprüche 1 bis 9, der
- 12 bis 30 Gew.-% radikalisch polymerisierbares Monomer (a),
- 6 bis 12 Gew.-%Ytterbiumtrifluoridpartikel (b),
- 40 bis 65 Gew.-% anorganischen Füllstoff (c),
- 15 bis 40% Kompositfüllstoff (d) und
- 0,1 bis 1 Gew.-% Initiator für die radikalische Polymerisation (e) enthält,
jeweils bezogen auf die Masse des Dentalwerkstoffs.

11. Dentalwerkstoff nach Anspruch 10, der
- 12 bis 30 Gew.-% radikalisch polymerisierbares Monomer (a),
- 3 bis 10 Gew.-% Ytterbiumtrifluoridpartikel (b) mit einer volumengemittelten Partikelgröße (D50-Wert) von 10 bis 24 nm,
- 45 bis 65 Gew.-% anorganischen Füllstoff (c),
- 15 bis 40 Gew.-% Kompositfüllstoff (d) und
- 0,01 bis 0,5 Gew.-% Initiator für die radikalische Polymerisation (e) enthält,
jeweils bezogen auf die Gesamtmasse des Dentalwerkstoffs.

12. Dentalwerkstoff nach Anspruch 10 oder 11, der zusätzlich bis zu 4 Gew.-%, vorzugsweise bis zu 3 Gew.-% Additiv(e) enthält, bezogen auf die Gesamtmasse des Dentalwerkstoffs.

13. Dentalwerkstoff nach einem der Ansprüche 1 bis 12, zur therapeutischen Anwendung als dentaler Zement, Beschichtungs- oder Verblendmaterial, vorzugsweise Füllungskomposit, besonders bevorzugt Bulk-Fill-Komposit.

14. Nicht therapeutische Verwendung eines Dentalwerkstoffs gemäß einem der Anspruch 1 bis 12 zur Herstellung von Inlays, Onlays, Kronen und Brücken.

## Claims

1. Dental material comprising
(a) 5 to 40 wt.-% of at least one radically polymerizable monomer,
(b) 1 to 30 wt.% of ytterbium trifluoride,
(c) 20 to 90 wt.% of at least one inorganic filler,
(d) 5 to 60 wt.% of at least one composite filler, and
(e) 0.005 to 3.0 wt.% of at least one initiator for the radical polymerization, preferably a photoinitiator,
in each case based on the mass of the dental material, **characterized in that**
the radically polymerizable **monomer (a)** comprises a mixture of
(a-1) 20 to 80 wt.% of urethane di(meth)acrylates, which comprise
- 5 to 60 wt.% of tetramethylxylylene diurethane dimethacrylate of formula V380, 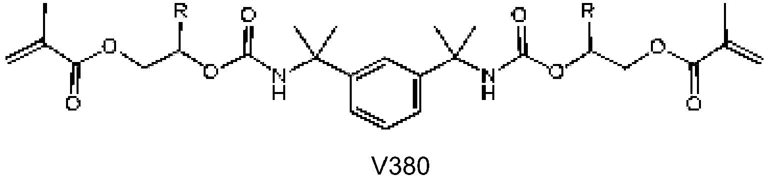 in which the radicals R are independently H or CH₃,
- 3 to 30 wt.-% of at least one difunctional urethane of formula 1, 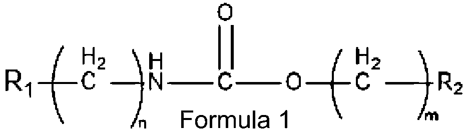 in which
R¹, R² = independently of one another are each H₂C=C(-R³)-C(=O)-O- or H₂C=C(-R⁴ )-C(=O)-NR⁵ -;
R³ = H or CH₃ ;
R⁴ = H or CH₃ ;
R⁵ = H or CH₃ ;
n, m = independently of one another are each an integer from 1 to 4, and
- 10 to 70 wt.-% of 1,6-bis-[2-methacryloyloxy-ethoxycarbonylamino]-2,2,4-trimethylhexane,
(a-2) 10 to 40 wt.-% of at least one radically polymerizable bisphenol A dimethacrylate,
(a-3) up to 40 wt.-% of at least one tricyclic dimethacrylate,
(a-4) up to 20 wt.% of other monomers, i.e., monomers that do not fall into any of the groups (a-1) through (a-3) and (a-5), and
(a-5) up to 8 wt.-% of at least one chain regulator,
in each case based on the total mass of component (a), wherein the monomer component (a) has a refractive index of 1.495 to 1.520,
the **ytterbium trifluoride (b)** has a volume-averaged particle size (D50 value) of ≤ 25 nm,
the inorganic **filler (c)** comprises glass powder with a refractive index in the range of 1.49 to 1.52 and a volume-averaged particle size (D50 value) of 0.4 to 0.9 µm, and
the **composite filler (d)** has an average particle size of 5 to 100 µm and has the following composition:
- 8 to 50 wt.% of a radically polymerizable monomer selected from di(meth)acrylates, alkylene dimethacrylates, urethane dimethacrylates, and mixtures thereof,
- 1 to 20 wt.% of ytterbium trifluoride particles having a volume-averaged particle size (D50 value) of ≤ 25 nm,
- 40 to 90 wt.% of additional inorganic filler, and
- 0.01 to 2 wt.% of an initiator for the radical polymerization,
in each case based on the total mass of the composite filler,
wherein the particle size is measured in all cases in the manner described in the description, and
wherein the refractive index of monomer component (a) corresponds to the refractive index of filler (c) or is at most 0.03 greater, and the refractive index of monomer component (a) corresponds to the refractive index of filler (d) or is at most 0.025 greater.

2. Dental material according to claim 1, which comprises
(a) 10 to 35 wt.% of at least one radically polymerizable monomer,
(b) 3 to 20 wt.% of ytterbium trifluoride having a volume-averaged particle size (D50 value) of 10 to 24 nm,
(c) 30 to 70 wt.% of at least one inorganic filler,
(d) 10 to 50 wt.% of at least one composite filler, and
(e) 0.01 to 2.0 wt.% of at least one initiator for the radical polymerization, preferably a photoinitiator,
in each case based on the mass of the dental material,
wherein the radically polymerizable **monomer (a)** comprises a mixture of
(a-1) 20 to 80 wt.% of urethane dimethacrylates, which comprise
- 5 to 60 wt.% of tetramethylxylylene diurethane dimethacrylate of formula V380,
- 3 to 30 wt.% of at least one difunctional urethane of formula 1, and
- 10 to 70 wt.-% 1,6-bis-[2-methacryloyloxy-ethoxycarbonylamino]-2,2,4-trimethylhexane,
(a-2) 10 to 40 wt.-% of at least one of bisphenol A dimethacrylate, 2,2-bis[4-(2-hydroxy-3-methacryloyloxypropyl)phenyl]propane, 2,2-bis[4-(2-meth-acryloxypropoxy)phenyl]propane, and 2-[4-(2-methacryloyloxyethoxy-ethoxy)phenyl]-2-[4-(2-methacryloyloxyethoxy)phenyl]propane),
(a-3) up to 40 wt.% of bis-(3-methacryloyloxymethyl)tricyclo-[5.2.1.0^{2,6} ]decane, and
(a-4) up to 20 wt.% of other monomers, i.e., monomers that do not fall into any of the groups (a-1) through (a-3) and (a-5), and
(a-5) up to 8 wt.-% of at least one chain regulator,
in each case based on the total mass of component (a),
the inorganic **filler (c)** comprises barium-free strontium glass powder having a refractive index in the range of 1.49 to 1.51 and a volume-average particle size (D50 value) of 0.4 to 0.9 µm, and
the **composite filler (d)** has an average particle size of 5 to 100 µm and has the following composition:
- 8 to 50 wt.% of a radically polymerizable monomer selected from glycerin dimethacrylate, 1,10-decanediol dimethacrylate, triethylene glycol dimethacrylate, 1,6-bis-[2-methacryloyloxy-ethoxycarbonylamino]-2,2,4-trimethylhexane, N-(2-methacryloyloxyethyl)carbamic acid (2-methacryl-oyloxyethyl) ester, tetramethylxylylene diurethane di(meth)acrylate, and mixtures thereof,
- 1 to 20 wt.% of ytterbium trifluoride particles having a volume-averaged particle size (D50 value) of 10 to 24 nm,
- 40 to 90 wt.% barium-free strontium glass powder, zircon silicate, and/or ZrO₂ particles, and
- 0.01 to 2 wt.% of an initiator for the radical polymerization,
in each case based on the total mass of the composite filler.

3. Dental material according to claim 1 or 2, wherein the radically polymerizable **monomer (a-1)** is selected from 1,6-bis-[2-methacryloyloxyethoxycarbonyl-amino]-2,2,4-trimethylhexane, N-(2-methacryloyloxyethyl)carbamic acid (2-methacryloyloxyethyl) ester, tetramethylxylylene diurethane dimethacrylate of formula V380, and 2-{[(2-(N-methylacrylamido)-ethoxy)-carbonyl]-amino}-ethyl methacrylate, the **monomer (a-2)** is selected from bisphenol A dimethacrylate, 2,2-bis[4-(2-hydroxy-3-methacryloyloxypropyl)phenyl]propane, ethoxy- or propoxylated bisphenol A dimethacrylate, 2-[4-(2-meth acryloyl oxy ethoxy ethoxy)phenyl]-2-[4-(2-meth acryloyl oxy ethoxy)phenyl]propane), and 2,2-bis[4-(2-methacryloxypropoxy)phenyl]propane, the **monomer (a-3)** is bis-(3-methacryloyloxymethyl)tricyclo-[5.2.1.0^{2,6}]decane, and the **monomer (a-4)** is 1,10-decanediol dimethacrylate.

4. Dental material according to any one of claims 1 to 3, which comprises, as a radically polymerizable monomer (a), a mixture of
(a-1) 20 to 80 wt.-%, preferably 30 to 70 wt.-%, and more preferably 40 to 67 wt.-% of the monomer mixture (a-1),
(a-2) 10 to 40 wt.-%, preferably 12 to 30 wt.-%, and more preferably 14 to 25 wt.-%, of at least one of bisphenol A dimethacrylate, 2,2-bis[4-(2-hydroxy-3-methacryloyloxypropyl)phenyl]propane and/or 2-[4-(2-meth-acryloyloxyethoxyethoxy)phenyl]-2-[4-(2-methacryloyloxy-ethoxy)phenyl]propane),
(a-3) optionally, up to 40 wt.-%, preferably 5 to 30 wt.-%, and more preferably 10 to 25 wt.-% of tricyclodecanedimethanol dimethacrylate, and
(a-4) optionally up to 20 wt.%, preferably 4 to 20 wt.%, and more preferably 4 to 10 wt.% of other monomers, i.e., monomers not falling into any of the groups (a-1) to (a-3) and (a-5), preferably 1,10-decanediol dimethacrylate,
(a-5) optionally up to 8 wt.-%, preferably 0.1 to 7 wt.-%, and more preferably 0.5 to 6 wt.-% of at least one chain regulator,
in each case based on the total mass of component (a).

5. Dental material according to any one of claims 1 to 4, wherein the variables in general formula 1 have the following meanings:
R¹ , R² = independently of one another are each H₂C=C(-R³ )-C(=O)-O- or H₂C=C(-R⁴ )-C(=O)-R⁵ -;
R³ = CH₃ ;
R⁴ = H;
R⁵ = CH₃ ;
n, m = independently of one another are each an integer from 1 to 2, and preferably 2.

6. Dental material according to claim 5, which comprises as component (a-1) a monomer mixture that comprises
- 10 to 45 wt.-% and preferably 10 to 25 wt.-% of tetramethylxylylene diurethane dimethacrylate of formula V380,
- from 3 to 25 wt.-% and preferably from 6 to 20 wt.-% of at least one difunctional urethane of formula 1,
- 15 to 60 wt.-% and preferably 20 to 47 wt.-% of 1,6-bis-[2-methacryloyloxyethoxycarbonylamino]-2,2,4-trimethylhexane,
in each case based on the total mass of the monomer component (a).

7. Dental material according to any one of claims 1 to 6, which comprises as X-ray-opaque filler (b) ytterbium trifluoride with a volume-averaged particle size (D50 value) of 10 to 24 nm.

8. Dental material according to any one of claims 1 to 7, wherein the inorganic filler (c) additionally comprises
- one or more zircon silicates having a volume-averaged primary particle size (D50 value) of 2 to 100 nm, more preferably 5 to 60 nm, and most preferably 10 to 40 nm, and/or
- ZᵣO₂ particles having a volume-averaged primary particle size (D50 value) of 0.5 to 50 nm, more preferably 1 to 20 nm, and most preferably 2 to 10 nm.

9. Dental material according to any one of claims 1 to 8, wherein the refractive index of the monomer component (a) corresponds to the refractive index of the filler (c) or is at most 0.002 to 0.02, preferably 0.005 to 0.015, greater, and wherein the refractive index of the monomer component (a) corresponds to the refractive index of the filler (d) or is at most 0.02, preferably at most 0.01, greater.

10. A dental material according to any one of claims 1 through 9, which comprises
- 12 to 30 wt.-% of radically polymerizable monomer (a),
- 6 to 12 wt.% of ytterbium trifluoride particles (b),
- 40 to 65 wt.% of inorganic filler (c),
- 15 to 40 wt.% of composite filler (d) and
- 0.1 to 1 wt.% of an initiator for the radical polymerization (e)
in each case based on the mass of the dental material.

11. Dental material according to claim 10, which comprises
- 12 to 30 wt.-% of radically polymerizable monomer (a),
- 3 to 10 wt.% of ytterbium trifluoride particles (b) with a volume-averaged particle size (D50 value) of 10 to 24 nm,
- 45 to 65 wt.% of inorganic filler (c),
- 15 to 40 wt.% of composite filler (d) and
- 0.01 to 0.5 wt.% of an initiator for the radical polymerization (e),
in each case based on the total mass of the dental material.

12. Dental material according to claim 10 or 11, which additionally comprises up to 4 wt.-%, preferably up to 3 wt.-%, of additive(s), based on the total mass of the dental material.

13. Dental material according to any one of claims 1 to 12, for therapeutic use as a dental cement, coating or veneering material, preferably as a filling composite, and more preferably as a bulk-fill composite.

14. Non-therapeutic use of a dental material according to any one of claims 1 to 12 for the manufacture of inlays, onlays, crowns, and bridges.

## Revendications

1. Matériau dentaire qui contient
(a) 5 à 40 % en poids d'au moins un monomère polymérisable par voie radicalaire,
(b) 1 à 30 % en poids de trifluorure d'ytterbium,
(c) 20 à 90 % en poids d'au moins une charge inorganique,
(d) 5 à 60 % d'au moins une charge composite, et
(e) 0,005 à 3,0 % en poids d'au moins un initiateur pour la polymérisation radicalaire, de préférence un photo-initiateur,
respectivement par rapport à la masse du matériau dentaire, **caractérisé en ce que**
le monomère polymérisable par voie radicalaire (a) contient un mélange de
(a-1) 20 à 80 % en poids d'uréthanes di(méth)acrylates qui contiennent
- 5 à 60 % en poids de tétraméthylxylylènediuréthanediméthacrylate de formule V380, 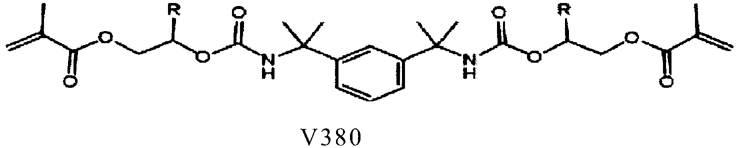 dans lequel les restes R sont H ou CH₃, indépendamment l'un de l'autre,
- 3 à 30 % en poids d'au moins un uréthane difonctionnel de formule 1 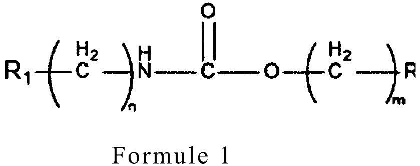 avec
R¹, R² = indépendamment l'un de l'autre, respectivement H₂C=C(-R³)-C(=O)-O ou H₂C=C(-R⁴)-C(=O)- NR⁵- ;
R³ = H ou CH₃ ;
R⁴ = H ou CH₃ ;
R⁵ = H ou CH₃ ;
n, m = respectivement un nombre entier de 1 à 4, indépendamment l'un de l'autre, et
- de 10 à 70 % en poids de 1,6-bis-[2-méthacryloyloxy-éthoxy-carbonylamino]-2,2,4-triméthylhexane,
(a-2) 10 à 40 % en poids d'au moins un diméthacrylate de bisphénol A polymérisable par voie radicalaire,
(a-3) jusqu'à 40 % en poids d'au moins un diméthacrylate tricyclique,
(a-4) jusqu'à 20 % en poids d'autres monomères, c'est-à-dire de monomères qui n'appartiennent pas à un des groupes (a-1) à (a-3) et (a-5), et
(a-5) jusqu'à 8 % en poids d'au moins un régulateur de chaîne,
respectivement par rapport à la masse totale du composant (a), le composant monomère (a) ayant un indice de réfraction allant de 1,495 à 1,520,
le trifluorure d'ytterbium (b) ayant une taille moyenne de particule en volume (valeur D50) de ≤ 25 nm,
la charge inorganique (c) contenant de la poudre de verre avec un indice de réfraction situé dans la plage allant de 1,49 à 1,52 et une taille moyenne de particule en volume (valeur D50) allant de 0,4 à 0,9 µm, et
la charge composite (d) ayant une taille moyenne de particule allant de 5 à 100 µm et présentant la composition suivante :
- 8 à 50 % en poids d'un monomère polymérisable par voie radicalaire, qui est choisi parmi des di(méth)acrylates, des alkylènes diméthacrylates, des uréthanes diméthacrylates et des mélanges de ceux-ci,
- 1 à 20 % en poids de particules de trifluorure d'ytterbium avec une taille moyenne de particule en volume (valeur D50) de ≤ 25 nm,
- 40 à 90 % en poids d'une autre charge inorganique, et
- 0,01 à 2 % en poids d'un initiateur pour la polymérisation radicalaire,
respectivement par rapport à la masse totale de la charge composite,
la taille de particule étant mesurée dans tous les cas de la manière indiquée dans la description, et
l'indice de réfraction du composant monomère (a) correspondant à l'indice de réfraction de la charge (c) ou étant supérieur d'au maximum 0,03 à celui-ci, et l'indice de réfraction du composant monomère (a) correspondant à l'indice de réfraction de la charge (d) ou étant supérieur d'au maximum 0,025 à celui-ci.

2. Matériau dentaire selon la revendication 1, qui contient
(a) 10 à 35 % en poids d'au moins un monomère polymérisable par voie radicalaire,
(b) 3 à 20 % en poids de trifluorure d'ytterbium avec une taille moyenne de particule en volume (valeur (D50) allant de 10 à 24 nm,
(c) 30 à 70 % en poids d'au moins une charge inorganique,
(d) 10 à 50 % d'au moins une charge composite, et
(e) 0,01 à 2,0 % en poids d'au moins un initiateur pour la polymérisation radicalaire, de préférence un photo-initiateur,
respectivement par rapport à la masse du matériau dentaire,
le monomère (a) polymérisable par voie radicalaire contenant un mélange de
(a-1) 20 à 80 % en poids d'uréthanes diméthacrylates qui contiennent
- 5 à 60 % en poids de tétraméthylxylylènediuréthanediméthacrylate de formule V380,
- 3 à 30 % en poids d'au moins un uréthane difonctionnel de formule 1, et
- 10 à 70 % en poids de 1,6-bis-[2-méthacryloyloxy-éthoxycar-bonylamino]-2,2,4-triméthylhexane,
(a-2) 10 à 40 % en poids d'au moins un composant parmi diméthacrylate de bisphénol A, 2,2-bis[4-(2-hydroxy-3-méthacryloy-loxypropyle)phényle]propane, 2,2-bis[4-(2-méthacryloxypro-poxy)phényle]propane et 2-[4-(2-méthacryloyloxyéthoxyé-thoxy)phényle]-2-[4-(2-méthacryloyloxyéthoxy)phényle]propane,
(a-3) jusqu'à 40 % en poids de bis-(3-méthacryloyloxyméthyle)tri-cyclo-[5.2.1.0^{2,6}]-décane, et
(a-4) jusqu'à 20 % en poids d'autres monomères, c'est-à-dire de monomères qui n'appartiennent pas à un des groupes (a-1) à (a-3) et (a-5), et
(a-5) jusqu'à 8 % en poids d'au moins un régulateur de chaîne, respectivement par rapport à la masse totale du composant (a),
la charge inorganique (c) contenant de la poudre de verre de strontium exempte de baryum, avec un indice de réfraction compris dans la plage allant de 1,49 à 1,51, et une taille moyenne de particule en volume (valeur D50) allant de 0,4 à 0,9 µm, et
la charge composite (d) ayant une taille de particule moyenne allant de 5 à 100 µm et présentant la composition suivante :
- 8 à 50 % en poids d'un monomère polymérisable par voie radicalaire, qui est choisi parmi le glycérinediméthacrylate, 1,10-décanedioldiméthacrylate, triéthylèneglycoldiméthacrylate, 1,6-bis-[2-méthacryloyloxy-éthoxycarbonylamino]-2,2,4-triméthylehexane, N-(2-méthacryloyloxyéthyl)acide carbamique-(2-méthacryloy-loxyéthyl)ester, tétraméthylxylylènediuréthanedi(méth) acrylate et des mélanges de ceux-ci,
- 1 à 20 % en poids de particules de trifluorure d'ytterbium avec une taille moyenne de particule en volume (valeur D50) allant de 10 à 24 nm,
- 40 à 90 % en poids de poudre de verre de strontium exempte de baryum, de silicate de zirconium et/ou de particules de Zr0₂, et
- 0,01 à 2 % en poids d'un initiateur pour la polymérisation radicalaire,
respectivement par rapport à la masse totale de la charge composite.

3. Matériau dentaire selon la revendication 1 ou 2, dans lequel le monomère (a-1) polymérisable par voie radicalaire est choisi parmi 1,6-bis-[2-méthacryloyloxy-éthoxycarbonylamino]-2,2,4-triméhtyl-hexane, N-(2-méthacryloyloxyéthyl)acide carbamique-(2-méthacryloy-loxyéthyl)ester, tétraméthylexylylènediuréthanediméthacrylate de formule V380 et 2-{[(2-(N-méthacrylamido)-éthoxy)-carbonyle]-amino=}-éythyleméthacrylate, le monomère (a-2) étant choisi parmi diméthacrylate de bisphénol A, 2,2-bis[4-(2-hydroxy-3-méthacryloy-loxypropyle)phényle]propane, diméthacrylate de bisphénol A éthoxylé ou propoxylé, 2-[4-(2-méthacryloyloxyéthoxyéthoxy)phényle]-2-[4-(2-méthacryloyloxyéthoxy)phényle]propane) et 2,2-bis[4-(2-méthacry-loxypropoxy)phényle]propane, le monomère (a-3) étant du bis-(3-mé-thacryloyloxyméthyle)tricyclo-[5.2.1.0^{2,6}]décane, et le monomère (a-4) étant du 1,10-décanedioldiméthacrylate.

4. Matériau dentaire selon une des revendications 1 à 3, qui contient, en tant que monomère (a) polymérisable par voie radicalaire, un mélange de
(a-1) 20 à 80 %, de préférence 30 à 70 % en poids, et de manière particulièrement avantageuse 40 à 67 % en poids du mélange de monomère (a-1),
(a-2) 10 à 40 % en poids, de préférence 12 à 30 % en poids, et de manière particulièrement avantageuse 14 à 25 % en poids d'au moins un composant parmi diméthacrylate de bisphénol A, 2,2-bis[4-(2-hydroxy-3-méthacryloyloxypropyle)phényle] propane et/ 2-[4-(2-méthacryloyloxyéthoxyéthoxy)phényle]-2-[4-(2-méthacryloyloxyéthoxy)phényle]propane,
(a-3) le cas échéant jusqu'à 40 % en poids, de préférence 5 à 30 % en poids, et de manière particulièrement avantageuse 10 à 25 % en poids de tricyclodécanediméthanoldiméthacrylate, et
(a-4) le cas échéant jusqu'à 20 % en poids, de préférence 4 à 20 % en poids, et de manière particulièrement avantageuse 4 à 10 % en poids d'autres monomères, c'est-à-dire de monomères qui n'appartiennent pas à un des groupes (a-1) à (a-3) et (a-5), de préférence 1,10-décanedioldiméthacrylate,
(a-5) le cas échéant jusqu'à 8 % en poids, de préférence 0,1 à 7 % en poids, et de manière particulièrement avantageuse de 0,5 à 6 % en poids d'au moins un régulateur de chaîne,
respectivement par rapport à la masse totale du composant (a).

5. Matériau dentaire selon une des revendications 1 à 4, pour lequel les variables de la formule 1 générale ont les significations suivantes :
R¹, R² = indépendamment l'un de l'autre, respectivement H₂C=C(-R³)-C(=O)-O- ou H₂C=C(-R⁴)-C(=O)-NR⁵- ;
R³ = CH₃ ;
R⁴ = H ;
R⁵ = CH₃ ;
n, m = indépendamment l'un de l'autre, respectivement un nombre entier de 1 à 2, et de préférence 2.

6. Matériau dentaire selon la revendication 5, qui contient, en tant que composant (a-1), un mélange monomère qui comporte
- 10 à 45 % en poids et de préférence 10 à 25 % en poids de tétraméthylxylylènediuréthanediméthacrylate de formule V380,
- 3 à 25 % en poids et de préférence de 6 à 20 % en poids d'au moins un uréthane difonctionnel de formule 1,
- 15 à 60 % en poids et de préférence de 20 à 47 % en poids de 1,6-bis-[2-méthacryloyloxyéthoxycarbonylamino]-2,2,4-trimé-thylehexane,
respectivement par rapport à la masse totale du composant monomère (a).

7. Matériau dentaire selon une des revendications 1 à 6, qui contient, en tant que charge (b) opaque aux rayons X, du trifluorure d'ytterbium avec une taille moyenne de particule en volume (valeur D50) allant de 10 à 24 nm.

8. Matériau dentaire selon une des revendications 1 à 7, dans lequel la charge inorganique (c) contient en outre
- un ou plusieurs silicates de zirconium avec une taille moyenne de particule en volume (valeur D50) allant de 2 à 100 nm, de manière particulièrement avantageuse de 5 à 60 nm, et de manière tout à fait préférable de 10 à 40 nm, et/ou
- des particules de ZrO2 avec une taille moyenne de particule en volume (valeur D50) allant de 0,5 à 50 nm, de manière particulièrement avantageuse de 1 à 20 nm, et de manière tout à fait préférable de 2 à 10 nm.

9. Matériau dentaire selon une des revendications 1 à 8, dans lequel l'indice de réfraction du composant monomère (a) correspond à l'indice de réfraction de la charge (c) ou est supérieur d'au maximum 0,002 à 0,02 à celui-ci, et de préférence de 0,005 à 0,015, et dans lequel l'indice de réfraction du composant monomère (a) correspond à l'indice de réfraction de la charge (d) ou est supérieur d'au maximum 0,02 à celui-ci, et de préférence au maximum de 0,01.

10. Matériau dentaire selon une des revendications 1 à 9, qui contient
- 12 à 30 % en poids de monomère (a) polymérisable par voie radicalaire,
- 6 à 12 % en poids de particules de trifluorure d'ytterbium (b),
- 40 à 65 % en poids de charge inorganique (c),
- 15 à 40 % en poids de charge composite (d), et
- 0,1 à 1 % en poids d'initiateur pour la polymérisation radicalaire (e),
respectivement par rapport à la masse totale du matériau dentaire.

11. Matériau dentaire selon la revendication 10, qui contient
- 12 à 30 % en poids de monomère (a) polymérisable par voie radicalaire,
- 3 à 10 % en poids de particules de trifluorure d'ytterbium (b) avec une taille moyenne de particule en volume (valeur D50) allant de 10 à 24 nm,
- 45 à 65 % en poids de charge inorganique (c),
- 15 à 40 % en poids de charge composite (d), et
- 0,1 à 0,5 % en poids d'initiateur pour la polymérisation radicalaire (e),
respectivement par rapport à la masse totale du matériau dentaire.

12. Matériau dentaire selon la revendication 10 ou 11, qui contient en outre jusqu'à 4 % en poids, de préférence jusqu'à 3 % en poids, d'additifs, par rapport à la masse totale du matériau dentaire.

13. Matériau dentaire selon une des revendications 1 à 12, destiné à une application thérapeutique en tant que ciment dentaire, matériau de revêtement ou de parement, de préférence composite de comblement, de manière particulièrement avantageuse composite de comblement en vrac.

14. Utilisation non thérapeutique d'un matériau dentaire selon une des revendications 1 à 12, aux fins de réalisation d'inlays, onlays, couronnes et bridges.
